# FASCICULE DE BREVET EUROPEEN

(11) **EP 2 988 608 B1**
(45) Date de publication et mention de la délivrance du brevet: **19.02.2020**
(21) Numéro de dépôt: 14711004.3
(22) Date de dépôt: 03.03.2014
(51) Int. Cl.: A23K 10/40, A61K 9/00, A61K 31/55, A61P 13/12

(54) **COMPOSITION ORALE NUTRITIONNELLE ET MEDICAMENTEUSE A USAGE VETERINAIRE**
ORALE NAHRUNGS- UND MEDIZINZUSAMMENSETZUNG ZUR VETERINÄRMEDIZINISCHEN VERWENDUNG
NUTRITIONAL AND MEDICINAL ORAL COMPOSITION FOR VETERINARY USE

(30) Priorité: 04.03.2013 FR 1351905
(43) Date de publication de la demande: 02.03.2016
(73) Titulaire: VIRBAC, 06516 Carros (FR)
(72) Inventeur: BRUNEL, Nicolas, F-06220 Vallauris (FR); MARTINS, Fanny, F-06800 Cagnes Sur Mer (FR); GOISNARD, Patricia, F-06300 Nice (FR)
(74) Mandataire: Nony
(86) Numéro de dépôt international: PCT/IB2014/059403
(87) Numéro de publication internationale: WO 2014/136035

(56) Documents cités:
- WO-A1-96/22028
- WO-A2-01/49272
- WO-A2-2010/030614
- US-A1- 2006 228 448

## Description

### DOMAINE DE L'INVENTION

La présente invention se rapporte au domaine des compositions orales à usage vétérinaire destinées à la prévention ou au traitement de troubles ou de pathologies chez les mammifères, en particulier les animaux de compagnie.

### ART ANTERIEUR

Les méthodes d'élevage d'animaux de ferme et les pratiques de maintien du bien-être des animaux de compagnie peuvent nécessiter l'administration à ces animaux des principes actifs médicamenteux.

Dans ces deux contextes relatifs au maintien de la santé animale, il est important que les principes actifs médicamenteux soient administrés en qualité et en quantité appropriées.

Pour les animaux de ferme, qui incluent les porcins, les ovins, les bovins, les caprins et les volailles, les médicaments, notamment les principes actifs antibiotiques, sont en général mélangés extemporanément aux aliments ou à l'eau. En effet, des aliments pour animaux de ferme prêts à l'emploi et comprenant des médicaments dans leur composition représenteraient une très faible proportion des aliments commercialisés. On ajoute que de tels aliments médicamenteux devraient être proposés pour une variété de principes actifs ou de combinaison de principes actifs, et pour une variété de combinaisons d'éléments nutritifs adaptés à chaque espèce d'animaux d'élevage. En conséquence les coûts de fabrication d'aliments médicamenteux pour animaux de ferme ne seraient pas compatibles avec les contraintes économiques de l'industrie agro-alimentaire. En général, les médicaments destinés aux animaux de ferme se présentent sous forme de granulés, lesquels sont mélangés extemporanément à la nourriture, par exemple au fourrage ou aux granulés industriels à base de céréales. Toutefois, ces pratiques ne permettent pas un contrôle précis de la quantité de médicament concrètement ingérée par les animaux, notamment parce que la distribution des granulés médicamenteux dans la masse du fourrage ou dans la masse des granulés de nourriture n'est pas homogène. A titre illustratif, les densités respectives distinctes des granulés alimentaires et des granulés médicamenteux entraînent un « dé-mélange », c'est-à-dire une distribution hétérogène des granulés médicamenteux dans la masse des granulés d'aliment, en raison notamment d'une décantation des granulés médicamenteux au sein du mélange de granulés. Les médicaments peuvent également être incorporés à l'eau de boisson, à condition que ceux-ci soient solubles dans l'eau et stables sous forme dissoute.

Pour les animaux de compagnie, en particulier pour les chiens et les chats, pour lesquels l'administration de principes actifs médicamenteux est réalisée de manière individuelle et non de manière collective, la pratique illustre souvent des difficultés liées à la mauvaise observance des traitements, en particulier des traitements par voie orale. On sait notamment que les médicaments présentés de manière séparée sous des formes solides, par exemple sous forme de pastilles, pilules, capsules, gélules, comprimés, sont difficilement acceptés par les animaux de compagnie, en particulier par les chiens et les chats, y compris lorsque ces formes solides sont mélangées à leur nourriture, et quelle que soit la forme de la nourriture, par exemple pâtée ou croquettes. La mauvaise observance des traitements médicamenteux par les animaux de compagnie est encore accrue lorsque le principe actif possède un goût prononcé, par exemple un goût amer, du fait du sens très développé de l'odorat et du goût de ces animaux.

L'incorporation d'un médicament dans l'alimentation des animaux implique que les caractéristiques de la composition finale nutritionnelle et médicamenteuse soient conformes aux exigences de la réglementation régissant la mise sur le marché des produits de santé. Une autorisation de mise sur le marché ne sera délivrée que si, notamment, il est établi avec certitude que la dose cible de principe actif est administrée à l'animal de façon exacte et reproductible. Le procédé de préparation de la composition doit donc être parfaitement maîtrisé.

Diverses solutions ont été proposées pour la fourniture de médicaments pour administration orale aptes à recueillir une bonne observance du traitement par les animaux.

Pour les animaux de ferme, spécifiquement, il a été proposé des granulés alimentaires revêtus d'un gel cohésif contenant le principe actif (voir demande PCT n° WO 96/22028).

Pour les animaux de compagnie, une variété de solutions a également été proposée, en particulier pour réduire les problèmes d'observance des traitements médicamenteux préventifs ou curatifs, tout particulièrement les problèmes d'observance des traitements médicamenteux par les chiens et les chats.

Par exemple, la demande de brevet européen n° EP 1 247 456 décrit des compositions pharmaceutiques sous forme de comprimés dans lesquelles le principe actif est mélangé à un agent d'appétence comprenant un ou plusieurs arômes naturels ou artificiels, notamment de la levure de bière, l'agent d'appétence étant destiné notamment à masquer le goût du principe actif. Une solution basée sur un principe similaire est divulguée dans la demande PCT n° WO 03/075895, laquelle décrit des comprimés, notamment à base de levure de bière, dans la masse desquels sont dispersées des particules composites comprenant le principe actif d'intérêt. Les particules composites sont formées d'un noyau de matériau support inerte qui est revêtu d'une couche contenant le principe actif, la couche de principe actif étant elle-même recouverte par une couche externe protectrice d'un matériau polymère destinée à masquer le goût du principe actif. Dans les comprimés décrits dans ce document, c'est la masse du comprimé dans laquelle sont présentes les particules de principe actif, par exemple la masse constituée de levure de bière, qui possède la fonction d'agent d'appétence. De plus, la technique mise en œuvre dans ce document nécessite la mise à disposition d'appareillages techniques complexes et coûteux.

Afin de surmonter les problèmes d'observance de traitements thérapeutiques par les animaux, la demande PCT n° WO 01/49272 propose des formulations de médicaments vétérinaires orales solides comprenant, au sein d'une masse moulée contenant des lipides et des agents aromatiques, des granules contenant le principe actif. Dans ces granules, le principe actif est micro-encapsulé dans une matrice polymère, en particulier une matrice d'éthylcellulose.

On a aussi décrit des compléments alimentaires dans lesquels est incorporé le principe actif d'intérêt.

On peut citer la demande PCT n° WO 01/35925 qui décrit des produits alimentaires vétérinaires comprenant un principe actif médicamenteux, et dans lesquels le goût dudit principe actif est masqué, ledit principe actif étant également protégé de la dégradation. Ce document décrit en particulier des micro-particules constituées d'alginate de calcium gélifié dans la masse desquelles est incorporé un principe actif d'intérêt, lesdites micro-particules étant elles-mêmes incluses dans un matériau contenant de la poudre de biscuit et de la farine, le mélange étant rendu cohésif du fait de la présence d'un agent liant.

De manière générale, les procédés de fabrication de croquettes pour animaux comprennent une étape d'obtention d'un noyau d'extrudé brut comprenant un mélange de substances nutritives (p. ex. protéines, lipides, glucides), lequel noyau d'extrudé est, lors d'une étape finale du procédé, revêtu d'une couche de graisse ou d'huile à chaud, en général par vaporisation ou atomisation de la graisse liquéfiée ou de l'huile chauffée sur la surface du noyau d'extrudé brut. Après refroidissement, le produit final est obtenu, lequel est un extrudé traité par application de graisse ou d'huile (aussi appelé « extrudé traité »). Lors de l'étape finale du procédé, le chauffage de la graisse ou de l'huile a pour fonctions principales (i) de liquéfier la graisse ou l'huile afin de permettre un revêtement de l'extrudé brut par vaporisation ou atomisation et (ii) le cas échéant stériliser la surface de l'extrudé brut par la chaleur et protéger la surface de l'extrudé brut d'une colonisation par des microorganismes pathogènes, tels que les bactéries pathogènes du genre *Salmonella.*

La préparation de croquettes pour animaux dont le noyau est revêtu d'une couche de graisse animale ou végétale selon le procédé général décrit ci-dessus est notamment illustrée dans la demande PCT n° WO 2012/099786. Selon ce procédé, la composition de graisse est d'abord liquéfiée par chauffage, par exemple à une température d'environ 125°C, puis la graisse liquide est atomisée sur le noyau des croquettes, puis les croquettes ainsi traitées sont convoyées vers un dispositif de refroidissement vibrant à lit d'air fluidisé. D'autres procédés d'obtention de croquettes pour animaux comprenant une étape d'application à chaud d'une couche de revêtement externe de graisse ou d'huile sont décrits notamment dans la demande de brevet n° US 2010/0303968 et dans les demandes PCT n° WO 2010/138372 et WO 2011/091111. De plus, de nombreux procédés connus comprennent une ultime étape de séchage des croquettes à haute température, par exemple à une température de 140°C, comme décrit par exemple dans la demande PCT n° WO 2011/091111 précitée.

Le type de procédé décrit ci-dessus, comprenant une étape finale d'enrobage à haute température d'un extrudé brut avec une graisse ou une huile liquéfiée, a aussi été utilisé dans l'état de la technique pour la fabrication de croquettes pour animaux comprenant, au sein de l'enrobage de graisse de l'extrudé enrobé final, un ou plusieurs additifs, dits additifs « actifs », susceptibles d'être bénéfiques pour la nutrition ou pour la santé de l'animal.

Une illustration d'un tel procédé d'obtention de croquettes comprenant un principe actif médicamenteux est exposée dans la demande PCT n° WO 2010/030614. Ce document décrit la fabrication de croquettes pour animaux possédant un noyau protéique (« extrudé brut »), dans lesquelles le noyau protéique est revêtu d'une couche additionnelle pouvant contenir un ou plusieurs additifs ou composants dits « actifs », tels que des sucres, des gommes, des protéines animales ou végétales, des vitamines, des acides gras, ou encore des agents biologiques tels que des microorganismes probiotiques et des enzymes. Ce document décrit en particulier un procédé de fabrication de croquettes comprenant un noyau à base de protéines végétales qui est revêtu d'une couche de graisse comprenant un microorganisme probiotique. Les noyaux à base de protéines végétales sont préparés dans une première étape du procédé. Puis la composition de revêtement du noyau est préparée en mélangeant par malaxage des microorganismes probiotiques avec une composition de graisse. Puis les noyaux sont revêtus d'une couche du mélange graisse / probiotiques en disposant les noyaux dans un mixeur à lit d'air fluidisé et en alimentant le mixeur à lit d'air fluidisé avec le mélange graisse / probiotiques qui a été préalablement liquéfié par chauffage, par exemple à une température de 56 °C. Après une étape de refroidissement, le produit final (aussi appelé « extrudé traité ») est obtenu. Toutes ces techniques impliquent une étape à haute température en présence de principes actifs, ce qui induit une dégradation plus ou moins importante du principe actif.

Il existe un besoin pour des compositions nutritionnelles et médicamenteuses vétérinaires orales, alternatives ou améliorées par rapport aux compositions connues, qui permettent d'incorporer de façon stable et efficace des principes actifs sensibles et ou amers et qui peuvent être fabriquées à un coût acceptable pour l'industrie de l'alimentation animale.

Il existe aussi un besoin pour des procédés améliorés pour la préparation de compositions nutritionnelles et médicamenteuses vétérinaires orales.

### RESUME DE L'INVENTION

La présente invention fournit un procédé pour l'obtention d'une composition orale nutritionnelle et médicamenteuse à usage vétérinaire comprenant les étapes suivantes :
a) fournir des noyaux d'extrudé d'aliment, et
b) enrober les noyaux d'extrudé d'aliment complet fournis à l'étape a) avec au moins une couche de matière grasse comprenant au moins un agent médicamenteux, ledit agent médicamenteux comprenant (i) au moins un principe actif pré-conditionné sous forme d'une solution ou d'une suspension dudit principe actif dans un liquide huileux ou (ii) au moins un principe actif pré-conditionné sous forme de granulés cireux, à une température inférieure à 40 °C.

Dans un autre mode de réalisation du procédé, l'étape b) d'enrobage des noyaux d'extrudé comprend les étapes suivantes :
b1) mettre en contact les noyaux d'extrudé d'aliment complet fournis à l'étape a) avec au moins un agent médicamenteux, ledit agent médicamenteux comprenant (i) au moins un principe actif pré-conditionné sous forme d'une solution ou d'une suspension dudit principe actif dans un liquide huileux ou (ii) au moins un principe actif pré-conditionné sous forme de granulés cireux, et
b2) enrober les noyaux d'extrudé obtenus à la fin de l'étape b1) avec au moins une couche de matière grasse, à une température inférieure à 40 °C.

Dans d'autres modes de réalisation du procédé, l'étape b) d'enrobage des noyaux d'extrudé comprend les étapes suivantes :
b3) obtenir une composition de matière grasse comprenant un agent médicamenteux, ledit agent médicamenteux comprenant (i) au moins un principe actif pré-conditionné sous forme d'une solution ou d'une suspension dudit principe actif dans un liquide huileux ou (ii) au moins un principe actif pré-conditionné sous forme de granulés cireux, et
b4) enrober les noyaux d'extrudé obtenus à la fin de l'étape a) avec au moins une couche de la composition de matière grasse comprenant ledit principe actif obtenue à la fin de l'étape b3), à une température inférieure à 40 °C.

Aux fins de la présente description, le terme « principe actif » désigne un principe actif de médicament admis par l'homme du métier, c'est-à-dire une substance entrant dans la composition d'un médicament et qui confère audit médicament ses propriétés thérapeutiques, préventives ou curatives.

Aux fins de la présente description, un principe actif pré-conditionné sous forme dissoute ou en suspension dans un liquide huileux ou sous forme de granulés cireux peut aussi être appelé « agent médicamenteux » ou « pré-mélange médicamenteux ».

La couche de matière grasse d'enrobage comprend au moins une matière grasse d'origine animale ou végétale.

Dans certains modes de réalisation du procédé, la couche de matière grasse est solide à une température inférieure à 25 °C. La graisse pourra alors être travaillée par malaxage ou mélange sans ajout de chaleur, pour obtenir une texture fluide apte à son utilisation comme agent d'enrobage. La couche de matière grasse peut comprendre au moins une matière grasse choisie parmi le saindoux, la graisse de porc, la graisse de bœuf, la graisse de canard ou la graisse de poisson.

Dans d'autres modes de réalisation, la couche de matière grasse est liquide à une température de 25 °C. La couche de matière grasse peut par exemple comprendre une huile de poisson.

Dans certains modes de réalisation du procédé, le(les) principe(s) actif(s), inclus dans l'agent médicamenteux, est(sont) sensible(s) à l'humidité.

Dans certains modes de réalisation du procédé, le principe actif est destiné à la prévention ou au traitement d'un trouble ou d'une maladie chronique ou d'une maladie nécessitant un traitement long et répétitif, par exemple quotidien. De telles maladies peuvent être des maladies chroniques hépatiques, rénales, vasculaires, digestives, inflammatoires, infectieuses ou encore des voies respiratoires, par exemple une insuffisance rénale, ou encore un cancer. Le principe actif peut ainsi être choisi parmi le métronidazole, la néomycine, les bronchodilatateurs comme l'aminophylline, les anti-inflammatoires non-stéroïdiens comme le carprofène, le meloxicam, le robenacoxib, le mavacoxib, le firocoxib, le deracoxib, la cyclosporine, la S-adenosyl-methionine, les beta-bloquants, les inhibiteur du récepteur à l'aldosterone, comme l'eplerenone ou la spironolactone, l'amlodipine, le pimobendan, le levosimendan, le torasemide, le furosemide, les alcaloïdes comme la théophylline, les antiangiogéniques... Dans le cas d'une insuffisance rénale en particulier, ledit principe actif peut être choisi parmi les inhibiteurs de l'enzyme de conversion de l'angiotensine (inhibiteurs de l'ACE), les inhibiteurs de la rénine et les antagonistes du récepteur de l'angiotensine II (composés en «-sartan» comme le losartan).

Les inhibiteurs de l'enzyme de conversion de l'angiotensine peuvent être choisis parmi le benazepril, l'enalapril, le ramipril, le quinapril, le preindopril, le lisinopril, l'imidapril, le zofnopril, le trandolapril, ainsi que les sels de ceux-ci.

L'invention fournit une composition orale nutritionnelle et médicamenteuse à usage vétérinaire, qui peut être aussi appelée aliment médicamenteux, comprenant un noyau d'extrudé d'aliment complet, ledit noyau étant enrobé d'au moins une couche de matière grasse, ladite couche de matière grasse comprenant au moins un principe actif sous forme de particules, ledit principe actif étant pré-conditionné sous forme dissoute ou en suspension dans un liquide huileux ou sous forme de granulés cireux.

La présente invention concerne aussi l'utilisation d'une composition telle que définie ci-dessus, pour la fabrication d'un alicament pour la prévention ou le traitement d'une maladie chronique ou nécessitant un traitement quotidien, par exemple de l'insuffisance rénale chez un mammifère non humain, en particulier le chien et le chat.

Elle a aussi trait à une composition de pré-mélange pour la fabrication d'une composition nutritionnelle et médicamenteuse comprenant une suspension huileuse de particules de bénazepril non-microencapsulé.

Il est aussi fourni selon l'invention une composition de pré-mélange médicamenteux pour la fabrication d'une composition nutritionnelle et médicamenteuse comprenant des particules de bénazepril non-microencapsulé réparties dans un milieu hydrophobe comprenant au moins une cire, sous forme de granulés cireux.

### DESCRIPTION DES FIGURES

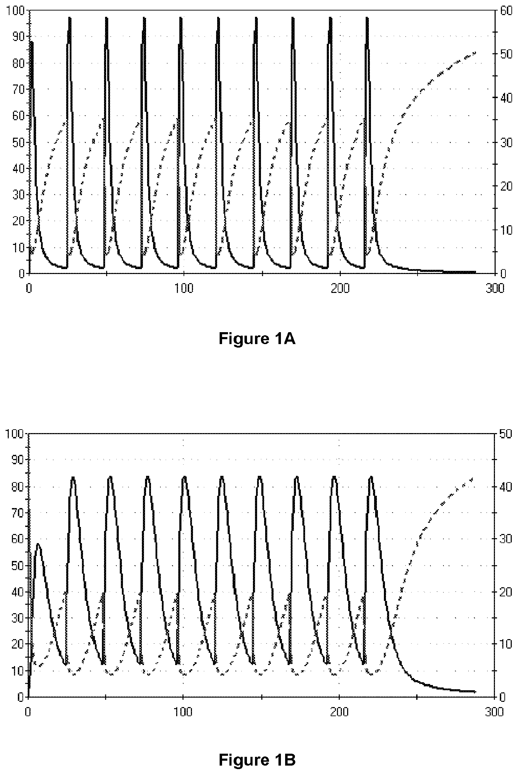

La **Figure 1** illustre les profils d'effet pharmacologique théoriques de :
   - figure 1A: comprimés de la spécialité pharmaceutique Fortekor®,
   - figure 1 B: composition orale vétérinaire décrite à l'exemple 1
   Sur les figures 1A et 1B, sont représentées (i) la courbe de concentration plasmatique de Bénazéprilate (métabolite plasmatique du Bénazepril) obtenue par modélisation et matérialisée par les courbes en trait plein, exprimée en ng/mL (ordonnée de gauche) et (ii) la courbe représentant le pourcentage d'inhibition de ACE (pour Enzyme de Conversion de l'Angiotensine) matérialisée par les courbes en pointillés, exprimée en pourcentage d'inhibition de ACE (ordonnée de droite).
   En abscisse : le temps après la première administration orale, exprimé en heures.

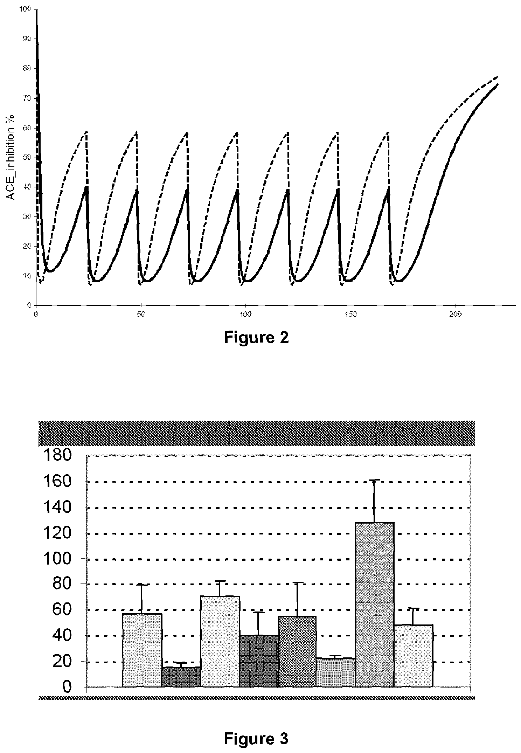

La **Figure 2** illustre la comparaison des effets pharmacologiques théoriques (i) de la composition orale vétérinaire décrite à l'exemple 1 (ligne continue) et (ii) de la spécialité pharmaceutique Fortekor® (ligne discontinue) à la même dose de 600µg/kg de bénazéprilate, sur l'inhibition de l'ACE plasmatique. En ordonnées : pourcentage d'inhibition d'ACE. En abscisses : le temps après la première administration orale, exprimé en heures.
La **Figure 3** illustre les Concentrations maximales (Cmax) moyennes (+/- SD) en bénazéprilate observées à J1 et J8 après traitement par Fortékor (colonne à carreaux) versus la composition selon l'invention (Inv) (colonne pleines) durant les études A et B. En ordonnées : Concentration plasmatique de Bénazéprilate, exprimée en µg/L. En abscisse, de gauche à droite : (1) Etude A, Fortekor Jour 1, (2) Etude A, Inv Jour 1, (3) Etude B, Fortekor Jour 1, (4) Etude B, Inv Jour 1, (5) Etude A, Fortekor Jour 8, (6) Etude A, Inv Jour 8, (7) Etude B, Fortekor Jour 8, (8) Etude B, Inv Jour 8.

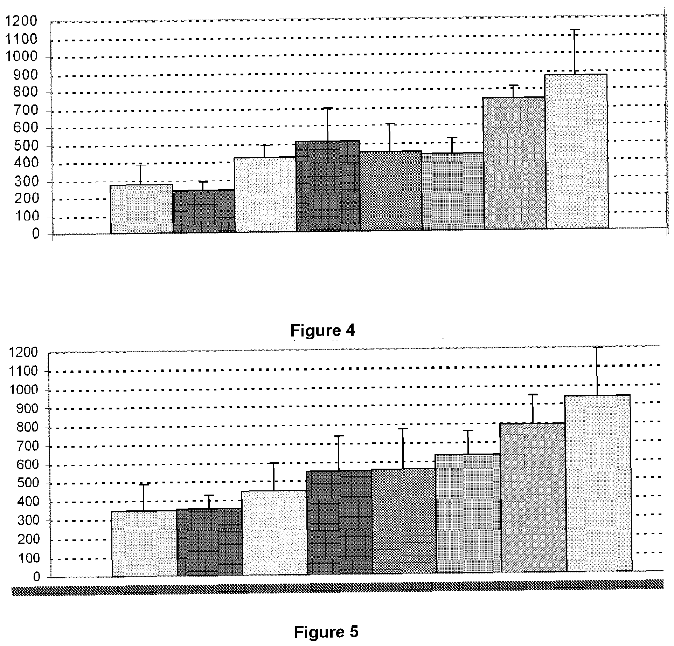

La **Figure 4** illustre l'aire sous la courbe des concentrations plasmatiques en bénazéprilate AUC last moyennes en bénazéprilate (+/- SD) à J1 et J8 après traitement au Fortékor versus la composition selon l'invention durant les études A et B. En ordonnées : Valeur de AUC Last moyen, exprimée en |ig.h/L de Bénazépril. En abscisse, de gauche à droite : (1) Etude A, Fortekor Jour 1, (2) Etude A, VIRBAC Jour 1, (3) Etude B, Fortekor Jour 1, (4) Etude B, Inv Jour 1, (5) Etude A, Fortekor Jour 8, (6) Etude A, Inv Jour 8, (7) Etude B, Fortekor Jour 8, (8) Etude B, Inv Jour 8.
La **Figure 5** illustre l'aire sous la courbe des concentrations plasmatiques en bénazéprilate (AUC last corr) moyennes (+/- SD) à J1 et J8 après traitement au Fortékor versus la composition selon l'invention durant les études A et B. En ordonnées : Valeur de AUC Last corr, exprimée en |ig.h/L de Bénazépril. En abscisse, de gauche à droite : (1) Etude A, Fortekor Jour 1, (2) Etude A, Inv En abscisse, de gauche à droite : (1) Etude A, Fortekor Jour 1, (2) Etude A, Inv Jour 1, (3) Etude B, Fortekor Jour 1, (4) Etude B, Inv Jour 1, (5) Etude A, Fortekor Jour 8, (6) Etude A, Inv Jour 8, (7) Etude B, Fortekor Jour 8, (8) Etude B, Inv Jour 8.

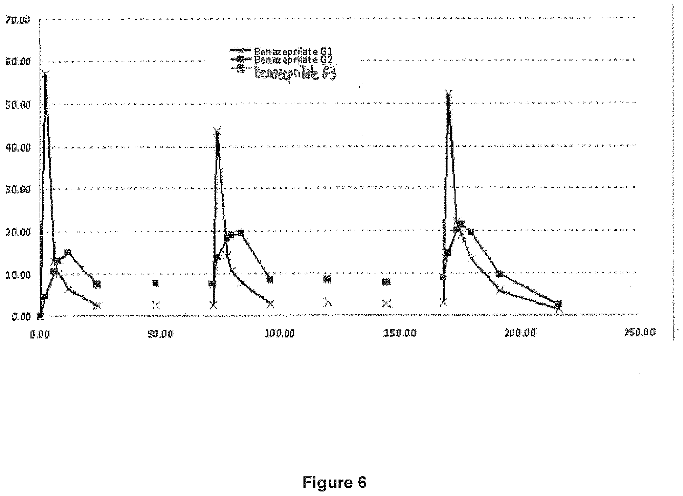

La **Figure 6** illustre une comparaison des profils pharmacocinétiques en Bénazéprilate après administration respectivement de la composition Inv (carrés) de la Composition Fortekor (croix). En ordonnées : Concentration plasmatique de Bénazéprilate, exprimée en ng/ml. En abscisse, profils pharmacocinétiques comparés de la composition Fortekor (courbes supérieures avec un pic étroit) et de la composition Inv (courbes inférieures avec un pic large), respectivement au Temps 0, au Temps 45 heures et au Temps 175 heures. On voit que le Cmax d'Inv est plus bas que le Cmax avec Fortékor, néanmoins les figures 4 et 5 montrent que les AUC sont comparables, conférant ainsi à Inv un profil pharmacologique meilleur sur le plan toxicologique, avec une efficacité comparable ou même supérieure.

### DESCRIPTION DETAILLEE DE L'INVENTION

La présente invention fournit un procédé de préparation d'une composition orale nutritionnelle et médicamenteuse, à usage vétérinaire.

La présente invention fournit aussi de nouvelles compositions vétérinaires orales qui sont à la fois nutritionnelles et médicamenteuses.

Il est apparu au demandeur qu'il existait un besoin pour des compositions destinées aux animaux qui incluent à la fois (i) les constituants nécessaires à une alimentation complète de ces animaux et (ii) un ou plusieurs principes actifs destinés à prévenir ou à traiter des troubles ou des pathologies qui pourraient affecter, ou qui affectent, lesdits animaux.

Le demandeur s'est donc attaché à la mise au point de compositions à usage vétérinaire pour administration orale comprenant des éléments nutritifs présents en une qualité et en une quantité adaptées à une alimentation complète des animaux, lesquelles compositions incluent aussi au moins un principe actif qui est présent en qualité ou en quantité adaptée pour la prévention ou le traitement d'un trouble ou d'une maladie chez lesdits animaux.

Pour parvenir à cet objectif, le demandeur s'est attaché à identifier des solutions qui permettent la coexistence, au sein d'une même composition orale, des substances nécessaires à la constitution d'un aliment complet, en particulier des substances contenant des lipides, des protéines ou des glucides, et au moins un principe actif, et dans lesquelles, notamment :
- le principe actif reste stable pendant une longue durée,
- la présence du principe actif dans la composition n'affecte pas l'appétence des animaux pour cette composition,
- la formulation choisie permet un masquage efficace du goût du principe actif lorsque ce principe actif possède un goût désagréable,
- le principe actif est distribué de manière homogène au sein de la composition, à la fois dans chaque unité de dosage et d'une unité de dosage à l'autre,
- le principe actif est libéré en qualité et en quantité appropriées pour l'obtention de l'effet pharmaceutique préventif ou curatif recherché, du fait notamment qu'il est inclus dans une composition nutritionnelle dont le temps de résidence dans l'estomac est long,
- les caractéristiques spécifiques de la composition ne requièrent pas la mise en œuvre d'un procédé de préparation complexe et/ou coûteux, ladite composition pouvant être préparée en utilisant une chaîne de fabrication industrielle d'un type connu
- le procédé mis en œuvre n'affecte pas de façon négative la stabilité du principe actif et sa teneur.

Après de longues recherches, le demandeur a mis au point un procédé de préparation de compositions orales nutritionnelles et médicamenteuses qui est de mise en œuvre simple et qui permet la fabrication de compositions ayant les propriétés avantageuses spécifiées ci-dessus.

La présente invention est relative à un procédé pour l'obtention d'une composition orale nutritionnelle et médicamenteuse à usage vétérinaire comprenant les étapes suivantes :
a) fournir des noyaux d'extrudé d'aliment complet, et
b) enrober les noyaux d'extrudé d'aliment complet fournis à l'étape a) avec au moins une couche de matière grasse comprenant au moins un agent médicamenteux, ledit agent médicamenteux comprenant (i) au moins un principe actif pré-conditionné sous forme d'une solution ou d'une suspension dudit principe actif dans un liquide huileux ou (ii) au moins un principe actif pré-conditionné sous forme de granulés cireux, à une température inférieure à 40 °C.

Les noyaux d'extrudé qui sont fournis à l'étape a) englobent (i) les noyaux d'extrudé comprenant un mélange de substances nutritionnelles qui n'ont pas été soumis à une étape de traitement final par application en surface de graisse ou d'huile liquéfiée à une haute température, soit dans un procédé « à chaud », lesquels noyaux sont aussi appelés noyaux « d'extrudé brut », et (ii) les noyaux d'extrudé préparés selon un procédé comprenant une étape d'application en surface de graisse ou d'huile liquéfiée chaude, lesquels noyaux sont aussi appelés noyaux « d'extrudé traités », aux fins de la présente description.

De manière surprenante, le demandeur a montré que l'incorporation « à froid » d'un agent médicamenteux tel que défini dans la présente description au sein d'une composition de graisse ou d'huile formant une couche d'enrobage d'un noyau d'extrudé d'aliment complet confère une grande stabilité dans le temps au principe actif qui est contenu dans ledit agent médicamenteux, c'est-à-dire à la fois (i) une protection du principe actif vis-à-vis d'une altération par l'environnement extérieur et (ii) une protection du principe actif vis-à-vis d'une altération provoquée par un contact de celui-ci avec les substances contenues dans le noyau d'extrudé. On comprend que cette caractéristique permet d'éviter, ou au moins de réduire substantiellement, la dégradation des principes actifs sensibles à l'hydrolyse.

En particulier, le fait que l'agent médicamenteux est incorporé « à froid » dans la couche d'enrobage de graisse ou d'huile, sans étape de liquéfaction par chauffage, préserve le principe actif contenu dans ledit agent médicamenteux de toute action de dégradation par la chaleur.

Egalement, le demandeur a montré que l'incorporation « à froid » de l'agent médicamenteux dans la couche d'enrobage de graisse ou d'huile évite, ou à tout le moins réduit substantiellement, une pénétration de la graisse ou de l'huile contenue dans la couche d'enrobage dans la masse du noyau d'extrudé. Cette caractéristique spécifique prévient l'éventuelle dégradation de tout ou partie du principe actif contenu dans l'agent médicamenteux du fait d'un contact de ce dernier avec le noyau d'extrudé. Cette caractéristique permet aussi, du fait de la présence de la totalité ou de la quasi-totalité de l'agent médicamenteux dans la couche d'enrobage, de contrôler le profil de libération du principe actif, dans l'objectif d'obtenir un effet pharmacologique optimal.

Il est ainsi montré que la combinaison particulière de caractéristiques de la composition orale vétérinaire ci-dessus permet une libération optimale du(des) principe(s) actif(s) d'intérêt, de nature à induire un effet médicamenteux préventif ou thérapeutique optimal. En particulier, il est montré qu'avec une composition orale vétérinaire telle que définie ci-dessus et administrée comme un aliment, on obtient un profil de libération du principe actif qui est du type à libération lente ou prolongée, ce qui permet le maintien de concentrations plasmatiques efficaces dudit principe actif pendant une durée plus longue qu'avec un médicament à libération immédiate et permet ainsi une activité globale accrue dudit principe actif sur les sites cibles de ce dernier dans l'organisme animal. Cela signifie qu'avec une composition orale vétérinaire conforme à l'invention, un effet pharmaceutique préventif ou curatif donné peut être obtenu avec une quantité moindre de principe(s) actif(s) qu'avec des compositions conventionnelles (AUC équivalente et Cmax inférieur au traitement de référence).

Il a aussi été montré selon l'invention que la composition orale nutritionnelle et médicamenteuse qui est obtenue à l'issue du procédé ci-dessus présente d'excellentes propriétés d'appétence pour les animaux, y compris lorsque le principe actif est amer ou repoussant pour l'animal.

Il est particulièrement important que l'aliment présente une excellente appétence. En effet, il est reconnu que les chats sont très sensibles au goût de leurs aliments et cet effet est encore exacerbé lorsqu'un chat est malade. Un effet secondaire courant de la maladie, notamment rénale, chez le chat, est la perte d'appétit. Il est donc crucial d'arriver à traiter efficacement un chat malade avant que celui-ci refuse complètement de s'alimenter. De plus, pour un traitement quotidien, il est très important de s'assurer d'une très bonne appétence, en effet, il faut éviter que l'animal n'associe l'aliment médicamenteux à un goût désagréable, ce qui rendrait l'observance désastreuse en quelques jours.

Par « noyau d'extrudé d'aliment complet », on entend selon l'invention la partie centrale de la composition orale qui est constituée par un mélange d'éléments nutritionnels, notamment lipides, protéines et glucides, en qualité et en quantité adaptées à l'alimentation des animaux auxquels la composition orale est destinée. En d'autres termes, une composition orale vétérinaire conforme à l'invention contient les éléments nutritifs essentiels pour les animaux auxquels elle est destinée.

La partie centrale ou noyau se présente sous la forme d'un produit issu de l'extrusion d'une matière première nutritionnelle ou d'un mélange de matières premières nutritionnelles, par exemple un mélange (i) de matières premières dérivées de céréales, (ii) de matières premières dérivées de viande, d'oeuf ou de poisson et (iii) de matières grasses d'origine animale ou végétale. L'utilisation d'un extrudé est tout à fait conventionnelle dans l'industrie agro-alimentaire, en particulier pour fabriquer les croquettes alimentaires destinées aux animaux, en particulier aux animaux de compagnie comme les chiens et les chats.

Comme cela a déjà été indiqué précédemment, un noyau d'extrudé englobe un noyau d'extrudé brut et un noyau d'extrudé traité, le noyau d'extrudé traité ayant été obtenu selon un procédé comprenant une étape d'application, en général par vaporisation ou atomisation, à haute température, d'une composition de graisse ou d'huile qui a été préalablement liquéfiée par chauffage.

Aux fins de la présente description, le terme « principe actif » désigne un principe actif de médicament admis par l'homme du métier, c'est-à-dire une substance entrant dans la composition d'un médicament et qui confère audit médicament ses propriétés thérapeutiques, préventives ou curatives.

Aux fins de la présente description, un principe actif (i) pré-conditionné sous forme d'une solution ou d'une suspension dudit principe actif dans un liquide huileux ou (ii) pré-conditionné sous forme de granulés cireux peut aussi être appelé « agent médicamenteux » ou « pré-mélange médicamenteux ». Un agent médicamenteux selon l'invention peut comprendre un seul principe actif ou bien une pluralité de principes actifs, par exemple 2, 3 ou 4 principes actifs distincts.

Dans certains modes de réalisation d'un agent médicamenteux comprenant une pluralité de principes actifs pré-conditionnés dans un liquide huileux, tous les principes actifs peuvent être dissous dans le liquide huileux.

Dans d'autres modes de réalisation d'un agent médicamenteux comprenant une pluralité de principes actifs pré-conditionnés dans un liquide huileux, tous les principes actifs peuvent être en suspension dans le liquide huileux.

Dans encore d'autres modes de réalisation d'un agent médicamenteux comprenant une pluralité de principes actifs pré-conditionnés dans un liquide huileux, au moins un des principes actifs est dissous dans le liquide huileux et au moins un autre des principes actifs est en suspension dans ledit liquide huileux.

Ainsi, dans la présente description, le terme « agent médicamenteux » désigne la forme physique dans laquelle est incorporé le principe actif, c'est-à-dire soit (i) sous forme d'une solution dudit principe actif dans un liquide huileux ou sous forme d'une suspension huileuse dudit principe actif, ou soit (ii) sous forme de granulés cireux, dans lesquels ledit principe actif médicamenteux est inclus dans une poudre d'une cire hydrophobe.

Selon l'invention, un principe actif peut être pré-conditionné par incorporation dudit principe actif dans une cire hydrophobe, c'est-à-dire principalement par incorporation d'un principe actif sous forme de particules de principe actif incluses dans une cire hydrophobe à une température comprise entre 15 °C et 25 °C, par exemple sous forme de granulés cireux. Le principe actif pré-conditionné sous forme de granulés cireux se présente avantageusement sous la forme d'une poudre de particules de cire hydrophobe dans lesquelles est réparti le principe actif. Préférentiellement, le principe actif pré-conditionné sous forme de granulés cireux se présente sous la forme d'une poudre de particules de cire hydrophobe dans lesquelles sont réparties des particules du principe actif.

### Procédé d'obtention d'une composition orale vétérinaire nutritionnelle et médicamenteuse.

A l'étape a), comme cela a déjà été précisé précédemment, les noyaux d'extrudé d'aliment complet sont avantageusement préparés selon des techniques conventionnelles, par exemple des techniques de préparation des matières premières, de mélange de celles-ci, puis d'extrusion, en particulier d'extrusion/cuisson, des mélanges obtenus, qui sont couramment utilisées dans le domaine de la fabrication de croquettes pour animaux, en particulier pour animaux de compagnie, y compris pour chiens ou chats.

Dans certains modes de réalisation du procédé, les noyaux d'extrudé, consistent en des noyaux « d'extrudé traité », lesquels sont préparés selon un procédé comprenant une étape de traitement d'un extrudé brut tel que décrit ci-dessus par application d'une graisse ou d'une huile préalablement liquéfiée par chauffage, comme décrit par exemple dans la demande PCT n° WO 2012/099786 ou encore dans la demande de brevet n° US 2010/0303968 et dans les demandes PCT n° WO 2010/138372 et WO 2011/091111.

Pour la préparation des noyaux d'extrudés, on peut utiliser des matières premières qui sont aisément accessibles dans le commerce.

Dans un autre mode de réalisation du procédé, l'étape b) d'enrobage des noyaux d'extrudé comprend les étapes suivantes :
b1) mettre en contact les noyaux d'extrudé d'aliment complet avec au moins un agent médicamenteux, ledit agent médicamenteux comprenant (i) au moins un principe actif pré-conditionné sous forme d'une solution ou d'une suspension dudit principe actif dans un liquide huileux ou (ii) au moins un principe actif pré-conditionné sous forme de granulés cireux, et
b2) enrober les noyaux d'extrudé obtenus à la fin de l'étape b1) avec au moins une couche de matière grasse, à une température inférieure à 40 °C.

Dans ces modes de réalisation du procédé, on met en contact les noyaux d'extrudé avec l'agent médicamenteux préalablement à l'étape d'enrobage avec la couche de matière grasse. Dans ces modes de réalisation, l'agent médicamenteux pénètre ultérieurement au sein de la couche de matière grasse d'enrobage, au sein de laquelle, notamment, le principe actif est protégé d'une détérioration par contact avec l'environnement extérieur.

L'étape b1) peut être réalisée par mélange de l'agent médicamenteux avec les noyaux d'extrudé, préalablement à l'étape d'enrobage avec la couche de matière grasse. Préférentiellement, l'étape b1) est réalisée à une température inférieure à 40 °C.

Dans d'autres modes de réalisation du procédé, l'étape b) d'enrobage des noyaux d'extrudé comprend les étapes suivantes :
b3) obtenir une composition de matière grasse comprenant au moins un agent médicamenteux, ledit agent médicamenteux comprenant (i) au moins un principe actif pré-conditionné sous forme d'une solution ou d'une suspension dudit principe actif dans un liquide huileux ou (ii) au moins un principe actif pré-conditionné sous forme de granulés cireux, et
b4) enrober les noyaux d'extrudé obtenus à la fin de l'étape a) avec au moins une couche de la composition de matière grasse comprenant ledit agent médicamenteux, obtenue à la fin de l'étape b3), à une température inférieure à 40 °C.

A l'étape b4) on enrobe les noyaux d'extrudés préférentiellement par malaxage ou mélange des noyaux d'extrudé, extrudé brut ou extrudé traité, avec une couche de matière grasse comprenant l'agent médicamenteux qui comprend le ou les principe(s) actif(s) d'intérêt. On utilise, préférentiellement un agent médicamenteux comprenant un principe actif médicamenteux qui se présente soit (i) sous forme d'une dissolution ou d'une suspension huileuse du ou des principe(s) actif(s), soit (ii) sous forme d'un pré-conditionnement du ou des principe(s) actif(s) dans un milieu hydrophobe, de préférence une cire, c'est-à-dire sous la forme de granulés cireux.

Quel que soit le mode de réalisation du procédé qui est mis en œuvre, l'étape b), d'enrobage des noyaux d'extrudé, notamment les étapes b1) et b2 ou les étapes b3) et b4), est préférentiellement réalisée à une température inférieure à 40 °C, mieux inférieure à 35 °C, préférentiellement inférieure à 30 °C, et de manière tout à fait préférée inférieure à 25 °C. A ces températures de réalisation de l'étape b), on n'observe pas d'altération du ou des principe(s) actif(s) ni des autres constituants de la composition orale vétérinaire. Notamment, on évite l'altération des protéines contenues dans le noyau d'extrudé d'aliment complet, la dégradation des vitamines et on évite aussi l'oxydation des graisses.

Selon un mode de réalisation préféré, l'ensemble du procédé selon l'invention est réalisé à une température telle qu'indiquée ci-dessus, à savoir inférieure à 40°C.

De manière générale, la composition de matière grasse utilisée pour l'étape b) d'enrobage comprend au moins une matière grasse d'origine animale ou végétale.

Comme cela a déjà été précisé et comme cela sera détaillé plus loin dans la présente description, le principe actif peut être pré-conditionné sous forme liquide, soit que le principe actif est dissous dans le liquide, soit que le principe actif est en suspension dans le liquide.

Dans d'autres modes de réalisation de l'agent médicamenteux, le principe actif est sous forme de particules et est pré-conditionné par incorporation dans une cire hydrophobe, c'est-à-dire sous forme de granulés cireux.

Le ou les principe(s) actif(s) constitutif(s) de l'agent médicamenteux utilisé à l'étape b) du procédé est(sont) défini(s) ailleurs dans la présente description.

### La composition de matière grasse d'enrobage

La ou les substance(s) lipidique(s) contenue(s) dans une couche de matière grasse d'enrobage de la composition orale vétérinaire peuvent être variées et incluent particulièrement les substances lipidiques d'origine naturelle, en particulier les matières grasses d'origine animale ou végétale.

Selon certaines variantes du procédé, la couche de matière grasse comprend au moins une matière grasse choisie parmi le saindoux, la graisse de porc, la graisse de bœuf, la graisse de canard ou la graisse de poisson.

Notamment, la matière grasse peut être d'origine variée. La matière grasse peut être choisie parmi du suif de bœuf, de la graisse de volaille, de l'huile de soja, de l'huile de canola, de l'huile de tournesol, de l'huile de poisson, du lard ou encore de la graisse blanche.

Les substances lipidiques peuvent notamment être choisies parmi :
(i) les substances lipidiques d'origine naturelle suivantes : cire d'abeille, lanoline, saindoux, graisse de porc, de bœuf ou de poisson, huile de poisson, cire de Carnauba, huile de soja, huile d'arachide, huile de colza, paraffine, cire microcristalline, vaseline, et une huile minérale, et
(ii) les substances lipidiques d'origine synthétique suivantes : alcool gras, acide gras, ester d'alcool et d'acide gras (cire blanche), huile végétale polyoxyéthylénée, huile végétale hydrogénée, mono-, di- et tri-glycérides d'acides gras.

Dans une variante préférée de l'invention, la substance lipidique d'origine naturelle est une huile minérale. Dans une autre variante préférée de l'invention, la substance lipidique d'origine synthétique est un alcool ou un acide gras ou un mélange de ceux ci.

Avantageusement, la majeure partie des substances lipidiques contenues dans la couche de matière grasse d'enrobage d'une composition orale vétérinaire conforme à l'invention possède un point de fusion supérieur à 30 °C, de préférence supérieur à 40 °C, et se présente donc sous forme solide aux températures habituelles de conservation des produits alimentaires ou pharmaceutiques.

De préférence, la couche de matière grasse d'enrobage se présente sous la forme d'une pâte qui peut être aisément travaillée à une température sensiblement inférieure à son point de fusion. La pâte de matière grasse est préférentiellement obtenue par malaxage, ou pommadage, de la substance lipidique ou de la combinaison de substances lipidiques constitutive de la matière grasse d'enrobage.

Dans certaines variantes de réalisation, le noyau d'extrudé d'aliment complet est enrobé d'une pluralité de couches de matière grasse, avec au moins une couche de matière grasse d'enrobage comprenant au moins un agent médicamenteux.

De manière générale, le nombre de couches de matière grasse d'enrobage varie de 1 à 5. Le plus souvent, une composition orale vétérinaire selon l'invention comprend une seule couche de matière grasse d'enrobage, laquelle comprend au moins un principe actif médicamenteux.

Dans ces modes de réalisation, la couche de matière grasse d'enrobage comprenant le ou les agent(s) médicamenteux consiste en la couche externe de la composition, ladite couche externe étant en contact avec l'environnement extérieur.

Dans les modes de réalisation ci-dessus, la couche de matière grasse d'enrobage comprenant au moins un agent médicamenteux peut consister en de la graisse animale, par exemple de la graisse de volaille, typiquement de la graisse de canard ou d'oie, ou bien encore du saindoux.

### Agent médicamenteux

Dans certains modes de réalisation d'une composition orale vétérinaire selon l'invention, le(s) principe(s) actif(s) d'intérêt compris dans l'agent médicamenteux se présente(nt) sous forme dissoute en solution huileuse, ou encore sous la forme d'une suspension dans un liquide huileux, notamment selon les caractéristiques de solubilité dudit (desdits) principe(s) actif(s).

Par solution huileuse ou liquide huileux, on entend une solution hydrophobe comprenant une huile ou un mélange d'huiles. Une huile peut être d'origine naturelle ou synthétique, en particulier une huile animale, végétale ou minérale.

Dans les modes de réalisation dans lesquels le principe actif est en suspension dans un liquide huileux, ledit liquide huileux peut comprendre, outre le véhicule huileux lui-même, également une ou plusieurs substances choisies parmi les agents viscosants, les agents de remise en suspension, les agents anti-oxydants, les agents conservateurs, les pigments, les agents d'arôme et les parfums.

Les agents viscosants peuvent être choisis parmi la silice, le mono, di et tristéarate d'aluminium et l'huile de ricin hydrogénée.

Les agents de remise en suspension peuvent être choisis parmi les agents tensioactifs amphiphiles, ce qui englobe les acides gras ou encore les lécithines.

Les agents anti-oxydants peuvent être choisis parmi le BHA, le BHT, le propyl gallate ou encore la vitamine E, ou un mélange de ces excipients.

Dans certains modes de réalisation avantageux d'un agent médicamenteux selon l'invention, le principe actif se présente sous la forme de particules.

Dans ces modes de réalisation, le principe actif l'agent médicamenteux peut être une suspension huileuse des particules de principe actif ou bien encore l'agent médicamenteux peut se présenter sous la forme de granulés cireux, dans lesquels sont réparties les particules de principe actif.

Dans ces modes de réalisation, le(s) principe(s) actif(s) d'intérêt sous forme de particules, se présente(nt) préférentiellement sous forme de micro-particules.

Avantageusement, la taille moyenne des micro-particules de principe actif est inférieure à 400 µm, ou à 100 µm, et mieux inférieure à 80 µm. La taille moyenne des particules de principe actif varie avantageusement de 10 µm à 50 µm, et va préférentiellement de 15 µm à 35µm. Pour mesurer la taille moyenne de particule de principe actif aux fins de la présente description, on utilise préférentiellement la technique par diffraction laser (« Low Angle Laser Light Scattering » ou LALLS) selon la Pharmacopée Européenne et notamment selon la PhEur 2.9.31 : Analyse de la taille des particules par diffraction de la lumière laser (norme ISO 13320 : 2009 & 9276.1), selon Ph. Eur. 2.9.35 Finesse des poudres mais aussi selon la méthode du tamisage analytique (Ph.Eur 2.9.38 ; ISO 3310.1), classification selon Ph.Eur. 2.9.12.

On précise que les particules de principe actif médicamenteux contiennent de manière tout à fait préférée seulement ledit principe actif, à l'exclusion de tout autre composé. Ces particules de principe actif non encapsulées sont donc dépourvues de couche d'un matériau empêchant un contact du ou des principe(s) actifs(s) avec l'environnement extérieur, et en particulier sont dépourvues d'une couche protectrice destinée à préserver le principe actif vis-à-vis d'une action destructrice de l'environnement extérieur. Les particules de principe actif sont en particulier dépourvues d'une couche permettant de masquer l'odeur ou le goût du principe actif.

Il est montré, de manière surprenante, que le caractère particulaire, de préférence micro-particulaire, du principe actif, combiné à l'absence de couche protectrice externe des particules ou micro-particules (non encapsulation), permet une bonne adhésion et un bon maintien du principe actif dans la couche hydrophobe qui n'est pas en contact avec le noyau d'extrudé d'aliment complet.

Les inventeurs ont montré que des avantages spécifiques sont obtenus lorsque le principe actif particulaire est inclus dans ladite couche de matière grasse d'enrobage sous la forme d'un agent médicamenteux, c'est-à-dire après un pré-conditionnement dans une substance lipidique, et plus spécifiquement lorsque le principe actif est pré-conditionné sous forme dissoute dans un liquide huileux ou sous forme d'une suspension huileuse ou sous la forme de granulés cireux.

L'utilisation du principe actif sous forme d'un pré-conditionnement sous forme d'un agent médicamenteux tel que défini dans la présente description (i) permet une grande stabilité du principe actif dans la composition orale vétérinaire et (ii) confère à la composition orale vétérinaire des propriétés d'appétence entraînant une meilleure observance de l'ingestion de la composition orale vétérinaire par les animaux. De plus, sur le plan pratique, il est plus aisé pour l'opérateur d'incorporer l'agent médicamenteux aux noyaux d'extrudés, par comparaison à l'incorporation d'un ou de plusieurs principes actifs qui ne sont pas pré-conditionnés sous forme d'un agent médicamenteux.

Ainsi, dans certains modes de réalisation d'une composition orale vétérinaire conforme à l'invention, l'agent médicamenteux comprend le ou les principe(s) actif(s) particulaires qui est (sont) pré-conditionné(s) sous forme d'une suspension huileuse.

Egalement, dans certains autres modes de réalisation d'une composition orale vétérinaire conforme à l'invention, l'agent médicamenteux comprend le ou les principe(s) actif(s) est (sont) pré-conditionné(s) sous forme de granulés cireux, dans lesquels sont incorporées les particules de principe(s) actif(s).

### Principes actifs

Du fait que les compositions orales vétérinaires de l'invention comprennent à la fois (i) au moins un agent médicamenteux et (ii) des nutriments en qualité et en quantité appropriées pour une alimentation complète des animaux, lesdites compositions orales vétérinaires sont adaptées à une utilisation pour la prévention ou le traitement de maladies, en particulier pour le traitement des maladies chroniques, qui nécessite une administration quotidienne de principe(s) actif(s) et sur une longue période de temps, par exemple pendant plusieurs mois ou plusieurs années, ou même durant toute la vie de l'animal.

Les maladies chroniques susceptibles d'être prévenues ou traitées avec une composition orale vétérinaire selon l'invention englobent les maladies chroniques hépatiques, rénales, vasculaires, digestives, inflammatoires, infectieuses ou encore des voies respiratoires.

Une composition orale vétérinaire conforme à l'invention est spécifiquement avantageuse lorsque la prévention ou le traitement d'une maladie nécessite à la fois (i) l'administration d'un ou plusieurs principe(s) actif(s) médicamenteux et (ii) l'application d'un régime alimentaire adapté.

Une composition orale vétérinaire telle que définie dans la présente description peut être utilisée notamment pour le traitement de l'insuffisance rénale.

Les principes actifs susceptibles d'être utilisés dans un agent médicamenteux constitutif d'une composition orale vétérinaire de l'invention englobent le métronidazole, la néomycine, les bronchodilatateurs comme l'aminophylline, les anti-inflammatoires non-stéroïdiens comme le carprofène, le meloxicam, le robenacoxib, le mavacoxib, le firocoxib, le deracoxib, la cyclosporine, la S-adenosyl-methionine, les beta-bloquants, les inhibiteurs du récepteur à l'aldosterone, comme l'eplerenone ou la spironolactone, l'amlodipine, le pimobendan, le levosimendan, le torasemide, le furosemide, les alcaloïdes comme la théophylline, les antiangiogéniques...

Dans le cas d'une insuffisance rénale en particulier, ledit principe actif peut être choisi parmi les inhibiteurs de l'enzyme de conversion de l'angiotensine (inhibiteurs de l'ACE), les inhibiteurs de la rénine et les antagonistes du récepteur de l'angiotensine II (composés en «-sartan» comme le losartan).

Les inhibiteurs de l'enzyme de conversion de l'angiotensine susceptibles d'être utilisés dans une composition orale vétérinaire de l'invention englobent le benazepril, l'enalapril, le ramipril, le quinapril, le preindopril, le lisinopril, l'imidapril, le zofnopril, le trandolapril, ainsi que les sels de ceux-ci.

Les inhibiteurs de la rénine susceptibles d'être utilisés dans une composition orale vétérinaire de l'invention englobent la pepstatine, les analogues peptidiques de la pepstatine tel que le H-142, les mimétiques peptidiques de la pepstatin tel que le remikiren et les mimétiques non peptidiques de la pepstatine tel que l'aliskiren, ainsi que les sels de ceux-ci.

Les antagonistes du récepteur à l'angiotensine II susceptibles d'être utilisés dans une composition orale vétérinaire de l'invention englobent le valsartan, le telmisartan, le losartan, l'irbesartan, l'azilsartan et l'omesartan, ainsi que les sels de ceux-ci.
Les principes actifs susceptibles d'être utilisés dans un agent médicamenteux constitutif d'une composition orale vétérinaire de l'invention englobent aussi le firocoxib, la cyclosporine, la S-adenosyl-methionine, l'eplerenone, la spironolactone, l'amlodipine et le levosimendan. Comme indiqué précédemment, une composition orale vétérinaire selon l'invention peut comprendre, à titre de principe actif médicamenteux, le bénazépril ou un sel de celui-ci. Une telle composition orale vétérinaire est utile dans la prévention ou le traitement de l'insuffisance rénale.

Selon certains modes de réalisation, une composition orale vétérinaire de l'invention utile pour les animaux affectés, ou susceptibles d'être affectés, par une insuffisance rénale comprend (i) du bénazépril et (ii) un noyau d'extrudé d'aliment complet dont la constitution qualitative et quantitative en nutriments est adaptée à un soutien de la fonction rénale en cas d'insuffisance rénale chronique, par exemple un noyau d'extrudé d'aliment complet ayant une faible teneur en phosphore ainsi qu'une faible teneur en protéines, lesdites protéines étant de qualité élevée.

Avantageusement, le bénazépril est utilisé sous forme de chlorhydrate de bénazépril.

Avantageusement, le bénazépril ou un sel de celui-ci est présent en une quantité variant de 0.0025 à 0.0100 % (25 à 100 ppm) en poids, par rapport au poids total de la composition orale vétérinaire. Par exemple, une composition orale vétérinaire selon l'invention peut comprendre environ 50 ppm en poids de bénazépril ou d'un sel de celui-ci, par rapport au poids total de ladite composition orale vétérinaire.

### Noyau d'extrudé d'aliment complet

Le terme « extrudé » est utilisé dans la présente description selon son sens conventionnel connu par l'homme du métier. L'extrudé d'aliment complet est le produit final d'un procédé comprenant une étape au cours de laquelle un mélange approprié de substances nutritives est passé au travers de la filière d'un dispositif extrudeur.

De manière générale, l'homme du métier peut aisément déterminer la constitution qualitative et quantitative appropriée en substances nutritives du noyau d'extrudé d'aliment complet contenu dans une composition orale vétérinaire conforme à l'invention, à partir de ses connaissances générales dans le domaine de la préparation des aliments pour animaux, y compris dans le domaine des aliments pour animaux de compagnie tels que les chiens et les chats, ce qui inclus les aliments sous forme de croquettes.

De manière générale, ledit noyau d'extrudé comprend des substances nutritives en qualité et en quantité appropriées, par exemple en qualité et en quantité définies selon des normes réglementaires, y compris des normes des autorités administratives sanitaires, telles que la Direction Générale de l'Alimentation rattachée au Ministère de l'Agriculture français, l'Autorité pour la Sécurité Alimentaire Européenne (« *European Food Safety Authority* » ou EFSA), le Centre pour le Médecine Vétérinaire (« *Center for Veterinarian Medicine* ») rattaché à la Food and Drug Administration des Etats-Unis, ou encore le « *American Feed Control Officiais Incorporated* » des Etats-Unis.

Les constituants utilisés pour préparer le noyau d'extrudé d'une composition orale vétérinaire conforme à l'invention incluent, sans y être limités, des substances farinacées, des substances protéiniques, et des matières grasses, sous forme de mélanges. Dans certains modes de réalisation, le noyau d'extrudé peut comprendre des protéines, des substances contenant de l'amidon, des substances contenant des fibres, des matières grasses, des substances minérales, des vitamines, sous forme de mélanges ou de combinaisons d'au moins deux de ces constituants.

Les substances protéiques englobent la viande, et les produits dérivés de la viande, de poulet, d'agneau ou de mouton, de dinde, de bœuf, de chèvre et de poisson. Les produits dérivés de la viande englobent les poumons, les reins, les foies, les estomacs et les intestins.

Les substances contenant de l'amidon englobent les substances dérivées des céréales, en particulier les substances dérivées du maïs, du blé, du riz, de l'avoine, ou encore du sorgho.

Les substances contenant des fibres englobent les fructo-oligosacharides, la pulpe de betterave, les mannane-oligosaccharides, la fibre d'avoine, la pulpe d'agrume, la carboxyméthyl cellulose, ainsi que les gommes telles que la gomme arabique, la gomme de guar, ou encore les marcs de pomme ou de tomate, et des mélanges ou des combinaisons de ceux-ci.

Les matières grasses englobent les matières grasses d'origine animale et les matières grasses d'origine végétale.

Les matières grasses d'origine animale englobent celles qui dérivent du poulet, de la dinde, du porc, du bœuf, ou encore de poisson, ainsi que des mélanges ou des combinaisons de celles-ci.

Les matières grasses d'origine animale englobent les huiles de poisson. Les matières grasses d'origine végétale englobent les huiles végétales telles que les huiles de maïs, les huiles de soja, les huiles de coton, les huiles de palme, les huiles de noix de coco, ainsi que les mélanges et les combinaisons de celles-ci.

Les substances minérales englobent le selenite de sodium, le phosphate monosodique, le carbonate de calcium, le chlorure de potassium, le sulfate ferreux, l'oxyde de zinc, le sulfate de manganèse, le sulfate de cuivre, l'oxyde de manganèse, l'iodure de potassium, le carbonate de cobalt, ainsi que les mélanges et les combinaisons de ceux-ci.

Les vitamines englobent le chlorure de choline, la vitamine E, l'acide ascorbique, l'acétate de vitamine A, le pantothénate de calcium, l'acide pantothénique, la biotine, le monoitrate de thiamine, la vitamine B12, la niacine, la riboflavine, l'inositol, le chlorhydrate de pyridoxine, la vitamine D3, l'acide folique, la vitamine C, ainsi que les mélanges et les combinaisons de celles-ci.

Le noyau d'extrudé d'aliment complet peut aussi comprendre des acides aminés tels que méthionine, leucine, lysine, tryptophane, arginine, cystéine, acide aspartique, taurine, ainsi que des mélanges et des combinaisons de ceux-ci.

Le noyau d'extrudé d'aliment complet peut comprendre des substances additionnelles tels que des agents antioxydants, des agents stabilisants, des agents liants, des agents épaississants, des agents exhausteurs de goût, des arômes, des agents conservateurs, des agents de charge, des agents émulsifiants, des édulcorants, des agents tampon, des colorants, des agents gélifiants et des agents humectants.

Les agents émulsifiants et/ou gélifiants englobent la gélatine, les éthers de cellulose, l'amidon, les esters d'amidon, les éthers d'amidon et les amidons modifiés. L'amidon englobe l'amidon non gélatinisé, l'amidon complètement gélatinisé et l'amidon partiellement gélatinisé, et des mélanges ou des combinaisons de ceux-ci. L'amidon partiellement ou totalement gélatinisé peut se présenter sous la forme d'amidon pré-gélatinisé, qui est hydraté au moment de l'emploi.

Notamment, le noyau d'extrudé d'aliment complet peut comprendre des substances additionnelles tels que des caroténoïdes, des polyphénols, des acides gras, des probiotiques, ou encore des prébiotiques.

Dans certains modes de réalisation d'une composition orale vétérinaire conforme à l'invention, la qualité et la quantité de substances nutritionnelles est adaptée à la physiologie spécifique des animaux affectés d'une insuffisance rénale, en particulier des chats souffrant d'une insuffisance rénale.

Dans ces modes de réalisation, ledit noyau d'extrudé comprend des lipides, des glucides et des protéines et possède une teneur réduite en phosphore et une teneur réduite en protéines, par rapport aux normes communément admises.

Dans certains modes de réalisation, on peut utiliser des noyaux d'extrudé d'aliment complet connus, dont la composition qualitative et quantitative en ingrédients est adaptée à un régime alimentaire destiné à des animaux, en particulier à des chats, affectés d'une insuffisance rénale chronique. On peut par exemple utiliser des noyaux d'extrudé qui sont décrits dans la demande PCT n° WO 2007/084986.

Dans ces modes de réalisation, ledit noyau d'extrudé peut par exemple comprendre (i) 5 % à 50 % en poids de protéines, (ii) de 0,001 % à 1 % en poids de sodium, et (iii) de 0,01 % à 1 % en poids de potassium, les pourcentages en poids étant exprimés par rapport au poids total dudit noyau d'extrudé. Dans ces modes de réalisation, ledit noyau d'extrudé peut par exemple comprendre (i) 8 % à 25 % en poids de protéines, (ii) de 0,05 % à 0,6 % en poids de sodium, et (iii) de 0,05 % à 0,9 % en poids de potassium, les pourcentages en poids étant exprimés par rapport au poids total dudit noyau d'extrudé. Dans ces modes de réalisation, ledit noyau d'extrudé peut par exemple comprendre (i) 10 % à 16 % en poids de protéines, (ii) de 0,1 % à 0,5 % en poids de sodium, et (iii) de 0,1 % à 0,5 % en poids de potassium, les pourcentages en poids étant exprimés par rapport au poids total dudit noyau d'extrudé. Dans ces modes de réalisation, ledit noyau d'extrudé peut par exemple comprendre (i) 5 % à 30 % en poids de protéines, (ii) de 0,01 % à 2 % en poids de sodium, (iii) de 0,01 % à 2 % en poids de potassium, et (iv) de 0,2 % à 1 % en poids de phosphore, les pourcentages en poids étant exprimés par rapport au poids total dudit noyau d'extrudé. Dans ces modes de réalisation, ledit noyau d'extrudé peut par exemple comprendre (i) 25 % à 50 % en poids de protéines, (ii) de 0,1 % à 2 % en poids de sodium, (iii) de 0,1 % à 2 % en poids de potassium, et (iv) de 0,2 % à 1 % en poids de phosphore, les pourcentages en poids étant exprimés par rapport au poids total dudit noyau d'extrudé.

Dans une variante, l'invention est plus spécifiquement adaptée au traitement ou à la prévention de l'insuffisance rénale. Dans ce cas, le noyau d'extrudé d'aliment complet comprend une quantité d'amidon d'au plus 25 % en poids, par exemple une quantité d'amidon variant de 20 % à 25 % en poids, par exemple une quantité d'amidon variant de 10 % à 15 % en poids, par rapport au poids total dudit noyau d'extrudé. Avantageusement, le noyau d'extrudé d'aliment complet comprend du phosphore et du sodium en quantités appropriées.

Avantageusement, ledit noyau d'extrudé comprend essentiellement, ou comprend exclusivement, des protéines d'origine animale.

Avantageusement, ledit noyau d'extrudé comprend aussi des vitamines, et en particulier une ou plusieurs des vitamines suivantes : vitamine A, vitamine D, vitamine E, vitamine K, vitamine B1, vitamine B2, vitamine B3, vitamine B6, biotine, acide folique et vitamine B12.

Avantageusement, ledit noyau d'extrudé comprend un ou plusieurs oligo-élément(s). En particulier, ledit noyau d'extrudé peut comprendre un ou plusieurs éléments additionnels choisis parmi le cuivre, le fer, l'iode, le manganèse, le magnésium, le sélénium et le zinc.

### Autres caractéristiques de la composition orale vétérinaire

Avantageusement, le noyau d'extrudé d'une composition orale vétérinaire selon l'invention représente de 75 % à 95 % en poids, par rapport au poids total de ladite composition.

Avantageusement, l'ensemble des couches de matière grasse enrobant ledit noyau d'extrudé représente de 5 % à 25 % en poids, par rapport au poids total de ladite composition, étant entendu que le poids du ou des agent(s) médicamenteux contenu(s) dans au moins l'une des couches de matière grasse est négligeable par rapport au poids total de la composition orale vétérinaire.

La couche de matière grasse d'enrobage comprenant le ou les agent(s) médicamenteux doit être présente en une quantité suffisante pour protéger le ou les principe(s) actif(s) contenus dans le(s)dit(s) agent(s) médicamenteux vis-à-vis d'un contact avec l'environnement extérieur, et en particulier vis-à-vis d'un contact avec de l'eau éventuellement présente dans l'environnement extérieur, y compris sous forme d'eau présente dans une atmosphère humide.

Dans certains modes de réalisation de la composition orale vétérinaire de l'invention, la couche de matière grasse d'enrobage comprenant le ou les principe(s) actif(s) représente de 4 % à 15 % en poids, par rapport au poids total de ladite composition. Dans ces modes de réalisation, la composition orale vétérinaire comprend une pluralité de couches de matière grasse, la ou les couche(s) de matière grasse autre(s) que la couche d'enrobage comprenant l'agent médicamenteux représentant avantageusement de 1 % à 10 % en poids, par rapport au poids total de ladite composition orale vétérinaire.

Dans des modes de réalisation avantageux, une composition orale vétérinaire conforme à l'invention, la couche de matière grasse d'enrobage contenant le ou les principe(s) actif(s) représente de 0.5 % à 15 % en poids, les pourcentages en poids étant exprimés par rapport au poids total de ladite composition orale vétérinaire. Dans ces modes de réalisation, la composition orale vétérinaire comprend une pluralité de couches de matière grasse, la ou les couche(s) de matière grasse autre(s) que le couche d'enrobage comprenant l'agent médicamenteux représentant avantageusement de 4 % à 10 % en poids, par rapport au poids total de ladite composition orale vétérinaire.

Dans les modes de réalisation de la composition orale vétérinaire de l'invention dans lesquels l'agent médicamenteux comprend au moins un principe actif sous forme d'une suspension huileuse de ce dernier, au moins une partie, sinon la totalité, de l'huile contenue dans la suspension huileuse de principe actif de départ est présente dans la couche de matière grasse d'enrobage comprenant ledit principe actif. Dans ces modes de réalisation spécifiques, la couche de matière grasse comprenant le principe actif peut comprendre de 0,05 % à 2 % en poids de l'huile provenant de la suspension huileuse de principe actif, par rapport au poids total de ladite composition orale vétérinaire.

Sans vouloir être lié par une quelconque théorie, le demandeur pense que, dans les modes de réalisation ci-dessus, les particules de principe actif sont, au sein de la couche de matière grasse d'enrobage qui les comprend, recouvertes partiellement ou totalement avec un film de l'huile provenant de la suspension huileuse de départ. Ledit film d'huile est de nature à renforcer encore la protection du principe actif vis-à-vis des actions délétères éventuelles provoquées par l'environnement extérieur. Par ailleurs, la présence dudit film d'huile à la surface des particules de principe actif est susceptible de contribuer aux caractéristiques du profil pharmacocinétique de la composition orale vétérinaire. Enfin, la présence dudit film d'huile à la surface des particules de principe actif pourrait aussi produire un effet de masquage du goût ou de l'odeur du principe actif vis-à-vis de la sensibilité olfactive ou gustative des animaux et ainsi renforcer les propriétés d'appétence de la composition orale vétérinaire de l'invention.

De plus, dans une composition selon l'invention, le principe actif est inclus dans une composition nutritionnelle ce qui entraîne que ladite composition possède un long temps de résidence dans l'estomac. Avec une composition selon l'invention, la vidange gastrique est substantiellement retardée par comparaison avec la vidange gastrique observée lors de l'ingestion d'une formulation pharmaceutique, y compris une formulation pharmaceutique à libération retardée. En conséquence, l'inclusion du principe actif dans la masse d'une composition comprenant un noyau d'extrudé d'aliment complet contribue substantiellement à l'obtention d'un profil pharmacocinétique qui est particulièrement approprié pour les principes actifs dont les sites cibles sont localisés dans l'intestin grêle, tels que les inhibiteurs de l'enzyme de conversion de l'angiotensine comme le bénazépril. A titre illustratif, il est connu que le temps de résidence gastrique d'un aliment sec, par exemple des croquettes, dans la cavité stomacale du chat est d'environ 14 heures à 16 heures. Par définition, un temps de résidence gastrique identique est attendu avec une composition orale nutritionnelle et médicamenteuse selon l'invention ce qui permet la libération de la totalité du principe actif contenu initialement dans cette composition dans l'estomac avant la survenue de la vidange gastrique, et assure ainsi une biodisponibilité optimale du principe actif pour les sites cibles. Ces caractéristiques d'une composition nutritionnelle et médicamenteuse selon l'invention sont tout particulièrement avantageuses pour les principes actifs dont l'effet pharmacologique est substantiellement accru lors d'une saturation des sites cibles par ces principes actifs.

Dans les modes de réalisation de la composition orale vétérinaire de l'invention dans lesquels le principe actif est incorporé sous forme de granules cireux, la couche de matière grasse comprenant le principe actif peut comprendre de 0,05 % à 2 % en poids de la cire, par rapport au poids total de ladite composition orale vétérinaire.

Avantageusement, le rapport pondéral entre la couche de matière grasse d'enrobage et l'agent médicamenteux sera compris entre 1/5 et 1/10, de préférence, environ de 1/7.

Dans ces modes de réalisation, le demandeur pense que la couche de cire enrobant les particules de principe actif a les fonctions décrites ci-dessus pour le film d'huile.

### Composition de pré-mélange ou de pré-conditionnement sous forme d'une suspension huileuse de principe actif

Comme cela est exposé dans la présente description, une première forme de départ préférentielle d'un principe actif, ou composition de pré-mélange préférentielle, utilisée pour la fabrication d'une composition orale vétérinaire conforme à l'invention est une suspension huileuse dudit principe actif. Il s'agit d'un mode de réalisation particulier d'un agent médicamenteux selon l'invention.

Pour la suspension huileuse, on utilise une huile dont le point de fusion est inférieur à 10 °C, ce qui inclut les huiles dont le point de fusion est inférieur à 9 °C, 8 °C, 7 °C, 6 °C, 5 °C, 4 °C, 3 °C, 2 °C, 1 °C ou 0 °C. En général, on utilise une huile dont le point de fusion est supérieur à -10 °C.

L'huile constitutive de la suspension huileuse peut comprendre, ou consister en, un triglycéride ou un mélange de triglycérides, ou encore d'un ester de polyol.

On peut par exemple utiliser un triglycéride d'acide caprique et/ou d'acide caprylique, qui est notamment commercialisé sous la dénomination Miglyol® 810 ou Miglyol® 812 par la Société Sasol (Allemagne).

On peut aussi utiliser des triglycérides d'acide caprique/acide caprique/acide linoléique, qui sont notamment commercialisés sous la dénomination Miglyol® 818 par la Société Sasol (Allemagne).

On peut aussi utiliser des triglycérides d'acide caprylique/acide caprique/acide succinique, qui sont notamment commercialisés sous la dénomination Miglyol® 829 par la Société Sasol (Allemagne).

On peut aussi utiliser une huile du type propylène glycol dicaprylate/dicaprate, qui est notamment commercialisée sous la dénomination Miglyol® 840 par la Société Sasol (Allemagne).

La suspension huileuse comprend au moins un principe actif, avantageusement à raison de 0,1 % à 10 % en poids, par exemple de 0,5 % à 5 % en poids, par exemple encore environ 1 % en poids, par rapport au poids total de ladite suspension huileuse.

La suspension huileuse de principe actif peut aussi comprendre un ou plusieurs agents de charge destiné(s) à contrôler la densité de ladite suspension de manière à permettre une distribution homogène des particules du principe actif dans la totalité du volume de la suspension, et de manière à éviter, ou à tout le moins réduire fortement, la sédimentation des particules du principe actif. Le viscosant, lorsqu'il est présent dans la suspension huileuse, représente avantageusement de 0,5 % à 3 % en poids, mieux de 1 % à 2 % en poids, et encore mieux environ 1,0 % en poids, par rapport au poids total de ladite suspension huileuse. A titre illustratif, le viscosant peut être du dioxyde de silice colloïdal comme par exemple de l'Aerosil® 200 (Evonik Industries).

La suspension huileuse de principe actif peut aussi comprendre au moins un agent tensio-actif, qui permet d'améliorer la remise en suspension. L'agent tensio-actif, lorsqu'il est présent dans la suspension huileuse, représente avantageusement de 0,1 % à 3,0 % en poids, mieux de 1 % à 2 % en poids, et encore mieux environ 1.5 % en poids, par rapport au poids total de ladite suspension huileuse. Par exemple, on peut utiliser un agent tensio-actif non ionique comme le Tween® 80 (Sigma Chemicals).

Ainsi, la présente invention concerne aussi une composition de pré-mélange, en particulier pour la fabrication d'une composition nutritionnelle et médicamenteuse à usage vétérinaire, comprenant une suspension huileuse de particules de principe actif, par exemple une suspension huileuse de bénazepril, non-microencapsulé.

### Composition de pré-mélange ou de pré-conditionnement sous forme de milieu hydrophobe, notamment une cire, dans laquelle est incorporé le principe actif

Comme cela est exposé dans la présente description, une seconde forme de départ préférentielle d'un principe actif, ou composition de pré-mélange préférentielle, utilisée pour la fabrication d'une composition orale vétérinaire conforme à l'invention consiste en une cire hydrophobe dans laquelle sont incorporées des particules de principe actif. Il s'agit d'un autre mode de réalisation particulier d'un agent médicamenteux selon l'invention.

Un tel milieu hydrophobe est préférentiellement obtenu selon des techniques de granulation cireuse, lesquelles sont bien connues de l'homme du métier.

La granulation cireuse des particules de principe actif peut être réalisée en lit d'air fluidisé, par malaxage ou encore par mélange.

L'étape de granulation cireuse peut être réalisée par pulvérisation d'une solution comprenant au moins un composé cireux et un solvant.

Dans certains modes de réalisation, la solution comprenant le composé cireux et le solvant peut aussi inclure au moins un viscosant.

Les solvants d'enrobage sont ceux classiquement utilisés par l'homme du métier. On peut citer à titre d'exemples l'eau, le chlorure de méthylène, l'éthanol, l'isopropanol et leurs mélanges.

Ce procédé est réalisé en lit d'air fluidisé, par malaxage, par mélange ou par tout autre procédé industriel similaire connu de l'homme du métier.

L'opération de séchage peut être réalisée en lit d'air fluidisé, en sécheur rotatif sous vide ou par toute technique équivalente permettant d'enlever les solvants résiduels.

Les composés cireux utilisés peuvent être notamment choisis dans le groupe constitué par: les cires, les cires Novata, les Gélucires (Gattefossé) et les Suppocires (Gattefossé), les macrogol glycériques, les acides gras (du type acide stéarique), les esters d'acides gras, le monostéarate de glycérol, les Précirol (Gattefossé), les Compritol (Gattefossé).

Parmi ces composés cireux, on utilisera avantageusement les composés cireux hydrophobes et encore plus avantageusement des composés cireux hydrophobes présentant un faible HLB (balance hydrophile-lipophile) et possédant un point de fusion compris entre 35 et 53 °C, préférentiellement entre 37 et 43 °C. On peut citer, à titre non limitatif les composés cireux commercialisés sous les noms de Gélucires 43/01 et de NovataAB.

Ces composés cireux peuvent être associés à du monostéarate de glycérol (GMS).

On peut utiliser des composés cireux hydrophobes présentant un faible HLB (balance hydrophile-lipophile) et possédant un point de fusion compris entre 35 °C et 53 °C, préférentiellement entre 37 °C et 43 °C en présence de lubrifiants. On peut citer, à titre non limitatif, Gélucire 43/01 et NovataAB, éventuellement associés à du monostéarate de glycérol (GMS).

Le composé cireux peut être aussi du palmitostéarate de glycérol, par exemple du Precirol® AT05 (Gattefossé).

La solution de départ comprend aussi avantageusement un agent de charge, tel qu'un sucre, ou encore un dérivé de sucre comme le mannitol ou le xylitol.

Dans la pâte produit final, le principe actif représente avantageusement de 0,1 % à 10 % en poids, mieux de 0,5 % à 5 % en poids, par exemple environ 1% en poids, par rapport au poids total de la composition de cire contenant celui-ci.

Avantageusement, le ou les composé(s) cireux sont présents en une quantité allant de 10 % à 50 % en poids, mieux de 15 % à 40 % en poids, par exemple de 20 % à 30 % en poids, ce qui comprend environ 25 % en poids, par rapport au poids total de la composition de cire contenant celui-ci.

Dans la composition de cire, le ou les agent(s) de charge peuvent être présents en quantité significative, par exemple à raison de plus de 50 % en poids, y compris jusqu'à au moins 75 % en poids, par rapport au poids total de ladite composition de cire. Le ou les agents de charges, lorsqu'ils sont présents, représentent moins de 80 % en poids, par rapport au poids total de la composition de cire contenant celui-ci.

Ainsi, la présente invention concerne aussi une composition de pré-mélange, en particulier pour la fabrication d'une composition nutritionnelle et médicamenteuse à usage vétérinaire, comprenant une suspension huileuse de particules de bénazépril non-microencapsulé.

La présente invention concerne aussi une composition orale telle que définie dans la présente description, pour son utilisation en tant que médicament à usage vétérinaire.

L'invention est aussi relative à une composition orale telle que définie dans la présente description, pour son utilisation pour la prévention ou le traitement d'un trouble ou d'une maladie chez un animal, en particulier chez un animal de compagnie, et spécialement chez le chien ou le chat.

L'invention a notamment trait à une composition telle que définie dans la présente description pour son utilisation pour la prévention ou le traitement de l'insuffisance rénale chez un mammifère non humain.

L'invention est également relative à l'utilisation d'une composition telle que définie dans la présente description, pour la fabrication d'un alicament pour la prévention ou le traitement de l'insuffisance rénale chez un mammifère non humain.

La présente invention décrit aussi une méthode pour prévenir ou traiter un trouble ou une maladie chez un animal, comprenant une étape au cours de laquelle, on administre audit animal, préférentiellement par voie orale, une quantité appropriée d'une composition orale vétérinaire telle que définie dans la présente description.

La présente invention est en outre illustrée par les exemples ci-après.

### Exemples

### Exemple 1 : Préparation d'une composition orale vétérinaire avec un principe actif pré-conditionné sous forme d'une suspension huileuse

### 1.1. Préparation du pré-mélange médicamenteux sous forme de suspension huileuse:

Le principe actif est le chlorhydrate de Bénazépril sous la forme d'une poudre ayant une taille moyenne de particules de 10 à 35 µm commercialisé par la Société Aurobindo (Inde).

On prépare une suspension huileuse à base de Miglyol®840 (Condea GmbH, Allemagne) à laquelle on ajoute de l'Aerosil®200 (dioxyde de silice colloïdale - 2 % poids/poids) et du Tween®80 (ester de polyéthylène et de sorbitol - 0,5 % p/p).

La poudre de principe actif est ajoutée à la suspension huileuse ci-dessus et la suspension huileuse est obtenue par homogénéisation par agitation à l'aide d'un agitateur de type Rotor Stator jusqu'à obtenir une suspension homogène. La concentration finale du principe actif dans la suspension huileuse était de 1,0% en poids, par rapport au poids total de la suspension huileuse.

### 1.2. Préparation de la composition orale vétérinaire

On fournit un extrudé d'aliment complet mono-composant du type RenalCat® commercialisé par la Société Virbac Nutrition sous la forme de croquettes qui ont été traitées par pulvérisation de graisse liquide à chaud (noyau d'extrudé traité).

Les noyaux d'extrudé traités sont enrobés par malaxage dans un mélangeur à pales du type pétrin, dans les conditions opératoires suivantes: introduction des noyaux d'extrudé traités, introduction du prémélange médicamenteux préparé comme décrit au §1.1. ci-dessus, et introduction d'une masse de matériau hydrophobe, de préférence de la graisse de canard, du saindoux ou de l'huile de poisson, dans un rapport de poids de pré-mélange médicamenteux/matériau hydrophobe de 1/7, mélange pendant 300 secondes maximum.

Dans chaque croquette de la composition finale, la couche hydrophobe contenant le principe actif représente environ 12 % en poids, par rapport au poids total de la croquette.

### Exemple 2 : Préparation d'une composition orale vétérinaire avec un principe actif pré-conditionné par incorporation dans une cire.

### 2.1. Préparation du principe actif pré-conditionné sous forme de granulés cireux

Le principe actif est le chlorhydrate de Bénazépril sous la forme d'une poudre ayant une taille moyenne de particules de 15 à 30 µm commercialisé par la Société Aurobindo (Inde).

On prépare des granulés cireux du principe actif par granulation sèche avec un mélange de Precirol® ATO 5 (distéarate de glycérol) et de Pearlitol® 160C (mannitol), à raison de 1 % (poids/poids) de chlorhydrate de bénazépril, 25 % (poids/poids) de Precirol® et 74 % (poids/poids) de Pearlitol® dans un dispositif granulateur du type Rotolab à double enveloppe chauffante muni d'outils de mélange (impeller) et d'émottage (chopper), dans les conditions opératoires suivantes : Température de consigne 50 à 80 °C, Température produit 50 à 70 °C, vitesse d'agitation entre 100 et 1000 rpm.

### 2.2. Préparation de la composition orale vétérinaire

On fournit un extrudé d'aliment complet du type Renal Cat ® commercialisé par la Société Virbac Nutrition sous la forme de croquettes qui ont été traitées par pulvérisation de graisse liquide à chaud (noyau d'extrudé traité).

Les croquettes d'extrudé traité sont enrobées par malaxage dans un mélangeur à pales du type Pétrin, dans les conditions opératoires suivantes: introduction des noyaux d'extrudé traités, introduction du prémélange médicamenteux préparé comme décrit au §2.1. ci-dessus, et introduction d'une masse de matériau hydrophobe, de préférence de la graisse de canard, du saindoux ou de l'huile de poisson, dans un rapport de poids pré-mélange médicamenteux/matériau hydrophobe de 1/7, mélange pendant 300 secondes maximum.

Les extrudés et la graisse de canard sont introduits, mélange 20 secondes puis on introduit le prémélange médicamenteux (et des autres composants tels que des vitamines), on mélange 90 secondes.

Dans chaque croquette de la composition finale, la couche hydrophobe contenant le principe actif représente environ entre 4,77 % (première variante ci-dessus) et 9.59 % (seconde variante ci-dessus)% en poids, par rapport au poids total de la croquette.

### Exemple 3 : Stabilité de la composition orale vétérinaire

Dans cet exemple, on a évalué les propriétés de stabilité des compositions orales vétérinaires telles que décrites dans les exemples 1 et 2.

### A. Matériels et Méthodes

Des croquettes, de chacune des compositions orales vétérinaires décrites à l'exemple 1 ou à l'exemple 2 sont conservées dans des sachets triple épaisseur (PE, OPP, AL) hermétiquement fermés.

Les sachets contenant les croquettes sont placés dans plusieurs enceintes dont la température et le taux d'humidité relative (HR) sont contrôlés.

Les conditions de stockage ont été les suivantes : (i) 5 °C ; (ii) 25 °C, 60 % HR ; (iii) 30 °C, 65 % HR et (iv) 40 °C, 75 % HR

La stabilité du principe actif contenu dans les croquettes a été évaluée après différentes durées de stockage dans les conditions indiquées ci-dessus. La stabilité du principe actif a été évaluée par mesure HPLC (Chromatogaphie liquide haute performance).

### Conditions opératoires

### Pré-mélange médicamenteux (Agent médicamenteux)

Colonne: Uptishère ODB (Longueur (cm) 25, DI (mm) 4,60, Greffage: C18, Granulométrie (µm): 5, T°C four à colonne : 25 °C, Débit : 0.8 ml/min, Injection : 20µl, Détection UV : 240 nm, Phase mobile: KH2PO4 5g/L (30 %): Méthanol (70%) Solvant de dilution: MeOH/H2O (70/30).

### Préparation des solutions

Solution analyse du pré-mélange selon l'example 1: Le produit est extrait avec un mélange isooctane/MeOH/H2O et microfiltré, la dilution est ajustée puis le produit est injecté dans la colonne.

Solution analyse du pré-mélange selon l'example 2: Le produit est extrait dans l'éthanol, complété à l'eau et microfiltré, la dilution est ajustée puis le produit est injecté dans la colonne.

### Aliment médicamenteux:

Colonne: Uptishère ODB (Longueur (cm) 25, DI (mm) 4,60, Greffage: C18, Granulométrie (µm): 5, T°C four à colonne : 25 °C, Débit : 0.8 ml/min, Injection : 40µl, Détection UV : 240 nm, Phase mobile: KH2PO4 5g/L (30 %): Méthanol (70 %) Solvant de dilution: MeOH/H2O (70/30).

Le produit est extrait avec du méthanol pur, la dilution est ajustée puis le produit est injecté dans la colonne.

**Tableau 1 : Stabilité du pré-mélange médicamenteux suspension I**

| | 1.5 mois | 3 mois | 6 mois | 12 mois |
|---|---|---|---|---|
| 5 °C | 99.6 | 100 | 99.8 | 98.8 |
| 25 °C/60 % HR | 99.0 | 99.9 | 98.4 | 97.0 |
| 30 °C/65 % HR | 98.5 | 99.0 | 98.3 | 96.8 |
| 40 °C/75 % HR | 100 | 99.4 | 99.2 | - |

**Tableau 2 : Stabilité de l'aliment médicamenteux fabriqué à partir de I**

| | 1.5 mois | 3 mois | 6 mois | 9 mois | 12 mois |
|---|---|---|---|---|---|
| 5 °C | 102.3 | 98.3 | 95.9 | 98.4 | 102 |
| 25 °C/60 % HR | - | - | 100.1 | 93.2 | - |
| 30 °C/65 % HR | - | - | 93.3 | 87.8 | 90.6 |
| 40 °C/75 % HR | 86.0 | 92.4 | 74.7 | - | - |

**Tableau 3 : Stabilité du pré-mélange médicamenteux granulé cireux II**

| | 1.5 mois | 3 mois | 6 mois |
|---|---|---|---|
| 25 °C/60 % HR | 100.7 | 100. | 98.9 |
| 40 °C/75 % HR | 97.9 | 97.5 | 92.7 |

**Tableau 4 : Stabilité de l'aliment médicamenteux fabriqué à partir de II**

| | 1.5 mois | 3 mois | 6 mois | 9 mois | 12 mois | 18 mois | 24 mois |
|---|---|---|---|---|---|---|---|
| 25 °C/60 % HR | 99.5 | 92.2 | 99.2 | 92.4 | 96.7 | 95.1 | 85.8 |
| 40 °C/75 % HR | 87.6 | 78.9 | 72.1 | - | - | - | |

### B. Résultats

Les résultats obtenus ont montré que :
- pour le pré-mélange médicamenteux conforme à l'exemple 1, la teneur en principe actif est de 99,2 % après 6 mois de conservation à 40 °C et 75 % d'humidité relative et est de 96,8 % après 12 mois de conservation à 30 °C et 65 % d'humidité relative.
- pour le pré-mélange médicamenteux conforme à l'exemple 2, la teneur en principe actif est de 98,9 % après 6 mois de conservation à 30 °C et 65 % d'humidité relative et est de 92,7 % après 6 mois de conservation à 40 °C et 75 % d'humidité relative.
- pour la composition orale vétérinaire conforme à l'exemple 1, la teneur en principe actif est de 98.4 % après 9 mois de conservation à 4 °C et est de 93.2 % après 9 mois de conservation à 25 °C et 60 % d'humidité relative.
- pour la composition orale vétérinaire conforme à l'exemple 2, la teneur en principe actif est de 94.9 % après 18 mois de conservation à 25 °C et 60 % d'humidité relative.

Les résultats montrent que les caractéristiques spécifiques d'une composition orale vétérinaire de l'invention permettent un haut niveau de stabilité au stockage, alors que les particules de principe actif ne sont pas enrobées par une couche protectrice vis-à-vis de l'humidité, ni micro-encapsulées, ce qui va à l'encontre de ce à quoi s'attend l'homme du métier, qui pense qu'une encapsulation est indispensable pour la conservation du principe actif.

### Exemple 4 : Propriétés d'appétence de la composition orale vétérinaire

Dans cet exemple, on a évalué les propriétés d'appétence de compositions orales vétérinaires obtenues selon les indications des exemples 1 et 2.

### A. Matériels et Méthodes

### A.1. Système d'essai

### A.1.1 Caractérisation

Les caractéristiques des chats sont indiquées ci-dessous.
Race : Européen
Nombre : 21
Sexe : 10 femelles et 11 mâles
Age : moyenne 2,9 ans ; min : 1.5 mois - max : 5.8 ans
Poids : 3,88 kg ; min : 2,28 kg - max : 5.45 kg
Identification Une carte d'identification était placée sur les portes des boxes d'hébergement des animaux mentionnant le code de l'étude, le numéro d'identifiant, la race, le sexe et la date de naissance du chat.

Les animaux étaient en bonne santé lors de leur inclusion et n'avaient pas reçu de traitement dans les 15 jours précédant le début de l'étude.

### A.1.2 Conditions d'hébergement et d'entretien

Les animaux sont restés dans leurs locaux habituels d'hébergement. L'entretien a été réalisé tous les jours sauf le dimanche. Les paramètres environnementaux ont été relevés tous les jours. Une photopériode de 12h de lumière et 12h d'obscurité a été maintenue. L'alimentation a été distribuée tous les jours sauf le dimanche. Etant un critère de l'étude, la distribution et le retrait de l'aliment sont repris dans le paragraphe « A.3.2 modalités d'administration ». L'eau était disponible *ad libitum.*

### A.1. 3 Constitution des groupes et acclimatation

Durant la semaine de test, les animaux ont été nourris individuellement le matin jusqu'à environ 16h. Afin de les habituer à ces conditions d'hébergement, ils ont été placés dans des cages individuelles dans les mêmes conditions la première semaine d'étude.

### A.2. Traitements

Les produits ont été distribués de manière randomisée. Le premier jour de test, chaque chat est placé dans un kennel le matin entre 7h et 9h. L'alimentation, 80 g de produit testé, était présentée à l'animal. Le comportement du chat était observé et reporté dans les fiches d'observations. Les gamelles étaient retirées entre 12h et 14h et les quantités restantes de croquettes étaient pesées.

Produits testés:
A : aliment de référence, Renal Cat ®
B : aliment médicamenteux avec granulation cireuse
C : aliment médicamenteux avec suspension, lot 1
D : aliment médicamenteux avec suspension, lot 2

### A.3. Déroulement de l'essai

### A.3.1 Plan d'expérience

L'étude s'est déroulée sur 4 semaines. Le premier jour, chaque chat est placé dans une cage individuelle entre 7h et 9h. L'alimentation, 80g du produit testé, était présentée à l'animal. Le comportement du chat était observé et reporté dans les fiches d'observations. Les gamelles étaient retirées entre 14h30 et 16h30 et les quantités restantes de croquettes étaient pesées.

### A.3.2. Modalités d'administration

La première semaine, tous les animaux ont été nourris avec de l'aliment Renal Cat ® (commercialisé par la Société Virbac Nutrition).

Puis, pour les semaines suivantes, le lundi, les animaux étaient tous nourris avec l'aliment de référence, le matin.

Du mardi au vendredi, les chats ont reçus 4 jours consécutifs d'administration randomisée de chacun des produits testés, y compris l'aliment de référence.

Du premier au dernier jour de test, la personne distribuant les croquettes appliquait le schéma suivant :

### Distribution :

1 : Peser 80g de croquettes dans un pot, noter la quantité exacte dans le cahier « alimentation des animaux ».
2 : Distribuer la gamelle avec les croquettes au chat
3: Observer l'animal lors de la distribution et compléter la fiche d'observation

### Retrait :

Entre 14h30 et 15h30, peser la gamelle (gamelles + croquettes restantes) et le reporter sur le cahier « alimentation des animaux »

### A.3.3 Signes cliniques

Une observation clinique a été effectuée lors de la distribution et le retrait des gamelles. Si un animal nécessitait un traitement, non prévu dans le cadre de cette étude, le directeur d'étude (vétérinaire) convenait avec le donneur d'ordre du traitement à mettre en place. Cependant, si un délai prolongé aurait entrainé une souffrance inacceptable pour l'animal, le directeur d'étude pouvait administrer le traitement approprié, le donneur d'ordre en étant informé dès que possible. Aucun des animaux en étude n'a nécessité de traitement en plus de ceux prévus.

### B. Résultats

### B.1 Consommations alimentaires

La première semaine d'administration de Renal Cat ® est considérée comme la semaine d'acclimatation des animaux à la nourriture et aux conditions d'hébergement. Les résultats obtenus lors de cette semaine ne sont pas utilisés pour la suite des calculs.

Les quantités moyennes ingérées par aliment sont présentées dans le tableau 5 ci-dessous.

**Tableau 5**

| Aliment | A | B | C | D |
|---|---|---|---|---|
| Quantité (g) | 50 | 50 | 48 | 50 |

### B.2. Conclusion

Vingt et un chats ont été alimentés pendant 4 semaines avec une composition orale vétérinaire selon l'exemple 1 ou l'exemple 2 ou avec l'aliment de référence Renal Cat®. L'aliment était distribué le matin et retiré entre 14h30 et 15h30. Les animaux ont été observés tous les jours. Pour toutes les formules testées quelques signes cliniques (vomissements) ont été relevés sans atteinte de l'état général des animaux.

### Tous les aliments démontrent une bonne appétence, attestant que l'incorporation du bénazépril dans l'aliment n'a pas modifié la prise alimentaire des chats.

### Exemple 5 : Modélisation du profil de libération du principe actif

On a évalué l'efficacité pharmacologique du principe actif contenu dans une composition orale vétérinaire conforme à l'invention, puis on a comparé les résultats de cette évaluation avec le niveau d'efficacité pharmacologique d'une formulation pharmaceutique connue contenant le même principe actif.

Le principe actif est le bénazéprilate, issu de la prodrogue le bénazépril tel que contenu dans les compositions orales vétérinaires décrites dans l'exemple 1.

On a comparé le profil de l'effet pharmacologique des compositions décrites dans l'exemple 1 au profil d'effet pharmacologique d'une formulation pharmaceutique de comprimés contenant des granules de bénazépril recouverts d'une couche de polymère, commercialisés sous la dénomination Fortekor®.

### A. Matériels et Méthodes

On a préparé trois groupes expérimentaux de six chats, respectivement :
- Groupe 1 : animaux témoins alimentés avec une composition similaire à celle décrite à l'exemple 1, mais qui ne contient pas de Bénazépril ;
- Groupe 2 : la composition orale vétérinaire décrite à l'exemple 1 contenant du bénazépril, et
- Groupe 3 : la composition connue de référence Fortekor®

Pour les chats du Groupe 2, ils ont été alimentés avec la composition orale vétérinaire décrite à l'exemple 1 et ont ingéré une quantité quotidienne de Bénazéprilate d'environ 600 µg/kg.

Pour les chats du Groupe 3, ils ont ingéré des comprimés de Fortekor® à raison d'une dose quotidienne de Bénazéprilate de 600 µg/kg. Les chats ont été alimentés immédiatement après l'ingestion des comprimés contenant le Bénazépril HCl.

Le traitement des chats de chacun des trois groupes a été réalisé pendant une durée de huit jours consécutifs.

On a prélevé régulièrement des échantillons de sang à partir de chaque chat de chacun des trois groupes pendant la totalité du test. Pour chaque échantillon de sang collecté, on a mesuré la quantité de Bénazéprilate par spectrométrie de masse en mode tandem (LC-MS/MS) Pour chaque échantillon de sang collecté, on a mesuré l'activité de l'enzyme de conversion de l'angiotensine (ACE). Le pourcentage d'inhibition de l'ACE a été calculé en utilisant le niveau sanguin d'activité ACE des chats du Groupe 1 comme référence (0 % d'inhibition).

Une analyse PK/PD sur la base du modèle physiologique décrit par Toutain et al (2000) a été conduite pour estimer la pharmacocinétique (PK) et la pharmacodynamie (PD) des deux groupes testés.

Les profils de l'effet pharmacologique (pourcentage d'inhibition de l'enzyme de conversion de l'angiotensine (ACE) en fonction du temps) ont été déterminés pour les diverses compositions en utilisant le modèle décrit par King et al. (2003, J Vet Pharmacol Ther, Vol. 26(3) : 213-224), comme cela est décrit ci-après.

### B. Résultats

Des courbes prédictives ont été obtenues par modélisation et sont représentées sur les figures 1 et 2.

Les résultats de la figure 1 montrent que le profil pharmacocinétique et l'effet pharmacologique (% d'inhibition de ACE) obtenus avec la composition orale vétérinaire sont très différents de ceux obtenus avec la composition Fortekor®. On voit un « effet retard » sur le groupe Inv (décalage sur le temps en heures des courbes pointillées par rapport au groupe Fortekor ®)

La figure 2 illustre la comparaison des courbes d'inhibition d'ACE, respectivement (i) avec la composition orale vétérinaire décrite à l'exemple 1 « Inv » (ligne continue) et (ii) avec la composition Fortekor® (ligne discontinue).

Les résultats de la figure 2 montrent que la composition Inv (moyenne globale de 75,14 % d'inhibition d'ACE) est substantiellement plus efficace que la composition de référence Fortekor® (moyenne globale de 59,62 % d'inhibition d'ACE).

### Exemple 6 : Etude comparative des profils pharmacocinétiques d'une composition selon l'invention (Inv) et de la composition commerciale Fortekor®.

Une étude comparative des données pharmacocinétiques (i) d'une composition orale vétérinaire préparée comme indiqué à l'exemple 1 (« Inv ») et (ii) de la composition commerciale Fortekor® a été réalisée.

L'analyse des données pharmacocinétiques a été réalisée avec le logiciel Kinetica 5.0.

### Résumé des caractéristiques de l'étude:

Référence : Fortekor 2,5mg comprimé (chlorhydrate de bénazépril).

Test: Inv 50mg chlorhydrate de bénazépril / kg (d'aliment), adm env. 0,67 mg de chlorhydrate de bénazépril/ kg 12 Chats, européen mâle (3,7 à 4,5 kg, 3,1 à 6,4 ans).
Administration orale pendant 8 jours
2 groupes en parallèle (2 x 6 chats)

La ration alimentaire est fixe à 70 g de Renal Cat ® seul ou 50 g Inv + 20 g Renal Cat ®
- Fortekor + (70 g) Renal Cat ® (repas spécifique)
- Inv (50g) + (si tout a été consommé) Renal Cat ® (20g)

Suivi de la consommation alimentaire (2h après l'administration du repas pour Fortekor et 0,5, 2 et 6h pour Inv)

Collecte du sang :
T0 à J-1
J1 puis à 2, 6, 8, 12 h après administration
J2, J3, J4, J5, J6, J7 et J8 avant l'administration du matin
J4 puis à 2, 6, 8, 12 h après administration
J8 puis à 2, 6, 8, 12h, 24h (J9) et 48h (J10)

Méthode d'analyse: LC/MS-MS, LOQ : 0.5 ng/mL pour le bénazépril et le bénazéprilate

### Suivi de la prise alimentaire des chats de l'expérimentation sur 8 repas successifs (J1 à J8) à 2 h (groupe Fortekor) ou 30 min., 2h, 6h et 24h (groupe Inv) après la distribution du repas.

Les résultats sont regroupés dans le tableau 6 ci-dessous:
Le suivi alimentaire a été réalisé 30 min, 2h, 6h et 24h après la distribution du repas pour tous les chats.

Ce tableau permet de souligner la très bonne appétence du produit selon l'Invention.

**Tableau 6**

| Sujet n° | Statut | Prise alimentaire après 2 h sur les 8 jours | Statut majoritairement observé | Prise alimentaire sur les J1 | Prise alimentair e sur les J8 |
|---|---|---|---|---|---|
| 1 | Groupe 1 - Fortekor® | 7 x tout consommé en 2h | VF et / ou F | VF et / ou F | S ou VS |
| | | 1 x reste 22 g après 2h | | | |
| 2 | Groupe 1 - Fortekor® | 6 x tout consommé en 2h | VF et / ou F | S | S ou VS |
| | | 2 x reste 3 & 15 g après 2h | | | |
| 3 | Groupe 1 - Fortekor® | 8 x tout consommé en 2h | VF et / ou F | VF ou F | VF ou F |
| 4 | Groupe 1 - Fortekor® | 8 x tout consommé en 2h | VF et / ou F | VF ou F | VF ou F |
| 5 | Groupe 1 - Fortekor® | 5 x tout consommé en 2h | * VF et / ou F | S ou VS | S ou VS |
| | | 3 x reste 15, 14 & 20 g après 2h | | | |
| 6 | Groupe 1 - Fortekor® | 3 x tout consommé en 2h | * Tendance à être S ou VS | S ou VS | VF ou F |
| | | 5 x reste 42, 40, 21, 18, & 17 g après 2h | | | |
| 7 | Groupe 2 - Inv | 8 x tout consommé en 0,5h | VF | VF | VF |
| 8 | Groupe 2 - Inv | 2 x tout consommé en 0,5h | F et VF | VF | F |
| | | 6 x tout consommé en 2h | | | |
| 9 | Groupe 2 - Inv | 8 x tout consommé en 0,5h | VF | VF | VF |
| 10 | Groupe 2 - Inv | 3 x tout consommé en 6h | VS et S | VS | S |
| | | 5 x tout consommé en 24h | | | |
| 11 | Groupe 2 - Inv | 8 x tout consommé en 0,5h | VF | VF | VF |
| 12 | Groupe 2 - Inv | 5 x tout consommé en 2h | F et S | S | F |
| | | 3 x tout consommé en 6h | | | |

| | | | | | |
|---|---|---|---|---|---|
| VF very fast, F fast, S slow, VS very slow. | | | | | |

### Tableau 6

### Concentrations plasmatiques de bénazépril:

**Tableau 7**

| | | Concentration en bénazépril (ng/ml) | |
|---|---|---|---|
| | Temps depuis le traitement | Fortekor | Inv |
| Jour 1 | T0 | Nd | Nd |
| | T2h | 8.48 +/- 10.50 | 5.19 +/- 2.55 |
| | T6h | Nd | 4.27 +/- 2.21 |
| | T8h | Nd | 3.48 +/- 2.29 |
| | T12h | nd | 3.02 +/- 1.58 |
| Jour 2 | T0 | Nd | Nd |
| Jour 3 | T0 | Nd | Nd |
| Jour 4 | T0 | Nd | Nd |
| | T2h | 8.08 +/- 5.06 | 4.54 +/- 2.28 |
| | T6h | Nd | 5.35 +/- 3.10 |
| | T8h | Nd | 4.07 +/- 3.00 |
| | T12h | Nd | 3.37 +/- 1.75 |
| Jour 5 | T0 | Nd | Nd |
| Jour 6 | T0 | Nd | Nd |
| Jour 7 | T0 | Nd | Nd |
| Jour 8 | T0 | Nd | Nd |
| | T2h | 7.21 +/- 2.56 | 5.21 +/- 2.17 |
| | T6h | 3.5 +/- 3.43 | 4.79 +/- 2.14 |
| | T8h | 2.42 +/- 2.75 | 3.55 +/- 1.79 |
| | T12h | 1.62 +/- 1.77 | 3.1 +/- 1.76 |
| | T24h | Nd | Nd |
| | T48h | Nd | Nd |

On observe des concentrations en bénazépril plus basses avec Inv par rapport à Fortekor®, mais elles sont maintenues pendant 8 à 12h.

### Comparaison des paramètres PK/PD

**Tableau 8**

| **Paramètres PK** | **J1** | **J4** | **J8** |
|---|---|---|---|
| **Cmax (ng/mL)** | | | |
| Fortekor | 7.67 ± 9.61 | 7.32 ±4.89 | 7.21 ± 2.56 |
| Inv | 5.44 ± 2.27 | 5.80 ± 2.94 | 5.49 ± 2.16 |

| **Tmax (h)** | | | |
|---|---|---|---|
| Fortekor | 2.00 ± 0.02 | 2.02 ±0.02 | 2.00 ± 0.00 |
| Inv | 3.34 ± 2.06 | 4.67 ±2.06 | 4.00 ± 2.19 |

| **AUClast (ng.h/mL)** | | | |
|---|---|---|---|
| Fortekor | 8.72 ± 9.33 | 10.79 ± 13.09 | 39.98 ± 31.09 |
| Inv | 45.80 ± 20.41 | 48.21 ± 26.78 | 46.78 ± 19.66 |

Cmax est plus élevé et plus variable dans le groupe 1 (Fortekor®) que dans le groupe 2 (Inv) et Tmax est plus faible dans le groupe 1 (Fortekor) que dans le groupe 2 (Inv). L'exposition en bénazépril est plus élevée après administration de Inv.

### Résultats concentration plasmatique de Bénazéprilate

Paramètres Pharmacocinétiques du bénazéprilate à J1, J4 et J8 pour les groupes 1 (Fortekor®) et 2 (Inv):

**Tableau 9**

| | **jour d'analyse** | | | |
|---|---|---|---|---|
| **Paramètre PK** | | | | **Ratio d'accumulation** |
| | J1 | J4 | J8 | |
| **Cmax (ng/mL)** | **Cmax 1** (n=5) | | **Cmax ss** | **=Cmax ss/Cmax 1** |
| Fortekor® | 57.16 ± 22.69 | 43.64 ±9.51 | 54.80 ±27.03 | 1.02 ± 0.49 |
| Inv | 15.72 ± 3.78 | 20.08 ± 3.42 | 22.23 ±2.82 | 1.47 ± 0.35 |
| **Cmin (ng/mL)** | **Cmin 1** | | **Cmin ss** | **= Cmin ss/ Cmin 1** |
| Fortekor® | 2.55 ± 1.31 | | 2.88 ± 0.92 | 1.20 ± 0.22 |
| Inv | 4.57 ± 1.49 | | 8.65 ± 3.25 | 2.02± 0.82 |
| **Tmax (h)** | | | | |
| Fortekor® | 2.00 ± 0.02 | 2.02 ±0.02 | 2.00 ± 0.00 | |
| Inv | 13.35 ± 5.49 | 9.66 ±2.65 | 7.38 ±3.29 | |
| **AUClast (ng.h/mL)** | | | | |
| Fortekor | 279.89 ± **107.70** | 270.23 ± **76.85** | 476.28 ± **172.44** | 1.44 |
| Inv | 242.11 ± **48.23** | 356.79 ± **46.93** | 504.48 ± **111.70** | 1.55 |

Au steady state, le Cmax a été 2 fois plus élevé dans le groupe Fortekor® par rapport au groupe Inv.

**Tableau 10 Paramètres Pharmacocinétiques (LC/MS-MS, LOQ 0.5 ng/mL)**

| **AUClast (ng.h/mL)** | **J1** | **J4** | **J8** |
|---|---|---|---|
| Fortekor® | 279.89 ± **107.70** | 270.23 ± **76.85** | 476.28 ± **172.44** |
| Inv | 242.11 ± **48.23** | 356.79 ± **46.93** | 504.48 ± **111.70** |

Les profils pharmacocinétiques moyens en Bénazéprilate sont représentés sur la Figure 6. Les profils cinétiques des concentrations moyennes en bénazéprilate sont différents après administration de Fortekor® et après administration de Inv. Les Cmax moyennes en bénazéprilate sont plus faibles, quelque soit le jour considéré, dans le groupe traité par Inv par rapport au groupe traité par Fortekor®.

Les concentrations en bénazéprilate mesurées avant chaque administration du comprimé, entre J2 et J8, sont plus élevées après administration de Inv qu'après administration de Fortekor®. On observe un meilleur maintien des concentrations minimales en bénazéprilate dans le groupe 2 (Inv) par rapport au groupe 1 (Fortekor®).

### Résultats et conclusions de l'essai

Les deux produits, Fortekor® et Inv, sont sous deux formes différentes : un comprimé et un aliment médicamenteux.

Les doses moyennes administrées de la forme de Fortekor et de Inv sont env. 600 µg/kg et environ 510µg/kg (valeurs moyennes à J1). La dose administrée sous forme de Fortekor est donc supérieure à la dose administrée sous forme de Inv.

### Observations sur les profils

Les résultats sont illustrés notamment dans les figures 3, 4, 5 et 6.

On est en présence de deux formulations différentes ayant deux profils différents:
- L'administration par voie orale du bénazépril (Fortekor®) sous la forme d'un comprimé produit un profil de type « adm bolus » avec un pic à 2 h (Tmax) atteignant une concentration maximale Cmax) oscillant entre 30 et 190 ng/mL suivi d'une décroissance des concentrations plasmatiques (voir Figure 3). On observe donc une disponibilité rapide et forte du bénazéprilate dans le plasma mais persistance courte dans le temps.
- L'administration par voie orale du bénazépril (Inv) sous la forme d'un aliment médicamenteux produit un profil de type « adm perfusion / retardée » avec une augmentation au cours du temps de la concentration pour atteindre un plateau (Tmax) entre 6 et 12h avec une concentration maximale (Cmax) oscillant entre 10 et 71 ng/mL suivi d'une décroissance lente des concentrations plasmatiques (voir Figure 4). On observe donc une disponibilité plus lente et moins forte du bénazéprilate dans le plasma avec une persistance plus longue et constante dans le temps.

La forme d'administration influence de manière significative le profil plasmatique en bénazéprilate chez le chat.

Après observation des données pharmacocinétiques et si l'on tient compte de la dose réellement administrée, l'administration du bénazépril sous forme d'aliment médicamenteux (Inv) conduit toujours à une AUC last et à une AUC last corr du bénazéprilate plus fortes que celles produites suite à l'administration du bénazépril sous forme d'un comprimé (Fortékor®), comme cela est illustré respectivement dans les figures 4 et 5. On peut donc en conclure que l'administration du produit selon l'invention est au moins aussi efficace que le traitement de référence, tout en proposant un produit très facile d'administration et donc permettant une excellente observance de traitement. On remarquera de plus que dans cet essai, 100 % des chats ont pris le comprimé Fortékor, par gavage, assuré par des vétérinaires habitués à cette manipulation alors que dans la pratique par contre, chez le propriétaire de chat, la prise effective du comprimé sera beaucoup plus aléatoire, dépendant du chat et de la dextérité du propriétaire, rendant les résultats d'exposition du produit encore moins bons pour Fortekor que pour le produit selon l'invention.

## Revendications

1. Procédé pour l'obtention d'une composition orale nutritionnelle et médicamenteuse à usage vétérinaire comprenant les étapes suivantes :
a) fournir des noyaux d'extrudé d'aliment complet, et
b) enrober les noyaux d'extrudé d'aliment complet fournis à l'étape a) avec au moins une couche de matière grasse comprenant au moins un agent médicamenteux,
ledit agent médicamenteux comprenant (i) au moins un principe actif pré-conditionné sous forme d'une solution ou d'une suspension dudit principe actif dans un liquide huileux ou (ii) au moins un principe actif pré-conditionné sous forme de granulés cireux, à une température inférieure à 40 °C.

2. Procédé pour l'obtention d'une composition orale nutritionnelle et médicamenteuse à usage vétérinaire comprenant les étapes suivantes :
a) fournir des noyaux d'extrudé d'aliment complet, et
b1) mettre en contact les noyaux d'extrudé d'aliment complet avec au moins un agent médicamenteux, ledit agent médicamenteux comprenant (i) au moins un principe actif pré-conditionné sous forme d'une solution ou d'une suspension dudit principe actif dans un liquide huileux ou (ii) au moins un principe actif pré-conditionné sous forme de granulés cireux, et
b2) enrober les noyaux d'extrudé obtenus à la fin de l'étape b1) avec au moins une couche de matière grasse, à une température inférieure à 40 °C.

3. Procédé selon la revendication 1, dans lequel l'étape b) comprend les étapes suivantes :
b3) obtenir une composition de matière grasse comprenant au moins un agent médicamenteux, ledit agent médicamenteux comprenant (i) au moins un principe actif pré-conditionné sous forme d'une solution ou d'une suspension dudit principe actif dans un liquide huileux ou (ii) au moins un principe actif pré-conditionné sous forme de granulés cireux, et
b4) enrober les noyaux d'extrudé obtenus à la fin de l'étape a) avec au moins une couche de la composition de matière grasse comprenant ledit principe actif obtenue à la fin de l'étape b3), à une température inférieure à 40 °C.

4. Procédé selon l'une des revendications 1 à 3, **caractérisé en ce que** la couche de matière grasse comprend au moins une matière grasse d'origine animale ou végétale.

5. Procédé selon l'une des revendications 1 à 4, **caractérisé en ce que** le principe actif médicamenteux est sensible à l'humidité.

6. Procédé selon l'une des revendications 1 à 5, **caractérisé en ce que** le principe actif médicamenteux est destiné à la prévention ou au traitement d'un trouble ou d'une maladie chronique.

7. Procédé selon l'une des revendications 1 à 6, **caractérisé en ce que** le principe actif médicamenteux est destiné à la prévention ou au traitement de l'insuffisance rénale.

8. Procédé selon l'une des revendications 1 à 7, **caractérisé en ce que** le principe actif médicamenteux sensible à l'humidité est choisi parmi les inhibiteurs de l'enzyme de conversion de l'angiotensine (inhibiteurs de l'ACE), les inhibiteurs de la rénine et les antagonistes du récepteur de l'angiotensine II, le firocoxib, la cyclosporine, la S-adenosyl-methionine, l'eplerenone, la spironolactone, l'amlodipine et le levosimendan.

9. Procédé selon la revendication 8, **caractérisé en ce que** les inhibiteurs de l'enzyme de conversion de l'angiotensine sont choisis parmi le benazepril, l'enalapril, le ramipril, le quinapril, le preindopril, le lisinopril, l'imidapril, le zofnopril, le trandolapril, ainsi que les sels de ceux-ci.

10. Procédé selon la revendication 8, **caractérisé en ce que** les antagonistes du récepteur à l'angiotensine II sont choisis parmi le valsartan, le telmisartan, le losartan, l'irbesartan, l'azilsartan et l'omesartan, ainsi que les sels de ceux-ci.

11. Procédé selon l'une des revendications 1 à 8, **caractérisée en ce que** le principe actif médicamenteux est le bénazépril ou un sel de celui-ci, sous forme particulaire, de préférence le chlorhydrate de bénazepril.

12. Composition orale nutritionnelle et médicamenteuse à usage vétérinaire comprenant un noyau d'extrudé d'aliment complet enrobé d'au moins une couche de matière grasse, ladite couche de matière grasse comprenant au moins un agent médicamenteux, ledit agent médicamenteux comprenant (i) au moins un principe actif pré-conditionné sous forme d'une solution ou d'une suspension dudit principe actif dans un liquide huileux ou (ii) au moins un principe actif pré-conditionné sous forme de granulés cireux.

13. Composition orale nutritionnelle et médicamenteuse à usage vétérinaire, **caractérisée en ce qu'**elle est obtenue par le procédé selon l'une des revendications 1 à 11.

14. Composition selon l'une des revendications 12 et 13, pour son utilisation en tant que médicament à usage vétérinaire.

15. Composition selon l'une des revendications 12 et 13, pour son utilisation pour la prévention ou le traitement de l'insuffisance rénale chez un mammifère non humain.

## Patentansprüche

1. Verfahren zum Erhalten einer oralen Nährstoff- und Arzneimittelzusammensetzung zur tierärztlichen Verwendung, umfassend die folgenden Schritte:
a) Bereitstellen von Kernen aus Alleinfuttermittelextrudat und
b) Überziehen der in Schritt a) bereitgestellten Kerne aus Alleinfuttermittelextrudat mit mindestens einer Fettschicht, umfassend mindestens ein medizinisches Mittel,
wobei das medizinische Mittel (i) mindestens einem Wirkstoff, der in Form einer Lösung oder einer Suspension des Wirkstoffs in einer öligen Flüssigkeit vorkonditioniert ist, oder (ii) mindestens einen Wirkstoff, der in Form wachsartiger Granulate vorkonditioniert ist, umfasst, bei einer Temperatur von weniger als 40°C.

2. Verfahren zum Erhalten einer oralen Nährstoff- und Arzneimittelzusammensetzung zur tierärztlichen Verwendung, umfassend die folgenden Schritte:
a) Bereitstellen von Kernen aus Alleinfuttermittelextrudat und
b1) Inkontaktbringen der Kerne aus Alleinfuttermittelextrudat mit mindestens einem medizinischen Mittel, wobei das medizinische Mittel (i) mindestens einem Wirkstoff, der in Form einer Lösung oder einer Suspension des Wirkstoffs in einer öligen Flüssigkeit vorkonditioniert ist, oder (ii) mindestens einen Wirkstoff, der in Form wachsartiger Granulate vorkonditioniert ist, umfasst, und
b2) Überziehen der am Ende von Schritt b1) erhaltenen Extrudat-Kerne mit mindestens einer Fettschicht bei einer Temperatur von weniger als 40°C.

3. Verfahren nach Anspruch 1, wobei Schritt b) die folgenden Schritte umfasst:
b3) Erhalten einer Fettzusammensetzung, umfassend mindestens ein medizinisches Mittel, wobei das medizinische Mittel (i) mindestens einem Wirkstoff, der in Form einer Lösung oder einer Suspension des Wirkstoffs in einer öligen Flüssigkeit vorkonditioniert ist, oder (ii) mindestens einen Wirkstoff, der in Form wachsartiger Granulate vorkonditioniert ist, umfasst, und
b4) Überziehen der am Ende von Schritt a) erhaltenen Extrudat-Kerne mit mindestens einer Schicht der am Ende von Schritt b3) erhaltenen, den Wirkstoff umfassenden Fettzusammensetzung bei einer Temperatur von weniger als 40°C.

4. Verfahren nach einem der Ansprüche 1 bis 3, **dadurch gekennzeichnet, dass** die Fettschicht mindestens ein Fett tierischen oder pflanzlichen Ursprungs umfasst.

5. Verfahren nach einem der Ansprüche 1 bis 4, **dadurch gekennzeichnet, dass** der medizinische Wirkstoff feuchtigkeitsempfindlich ist.

6. Verfahren nach einem der Ansprüche 1 bis 5, **dadurch gekennzeichnet, dass** der medizinische Wirkstoff zur Vorbeugung oder Behandlung einer chronischen Störung oder Krankheit bestimmt ist.

7. Verfahren nach einem der Ansprüche 1 bis 6, **dadurch gekennzeichnet, dass** der medizinische Wirkstoff zur Vorbeugung oder Behandlung von Niereninsuffizienz bestimmt ist.

8. Verfahren nach einem der Ansprüche 1 bis 7, **dadurch gekennzeichnet, dass** der feuchtigkeitsempfindliche medizinische Wirkstoff aus Inhibitoren des Angiotensin-Converting-Enzyms (ACE-Inhibitoren), Renin-Inhibitoren und Angiotensin-II-Rezeptor-Antagonisten, Firocoxib, Ciclosporin, S-Adenosylmethionin, Eplerenon, Spironolacton, Amlodipin und Levosimendan ausgewählt ist.

9. Verfahren nach Anspruch 8, **dadurch gekennzeichnet, dass** die Inhibitoren des Angiotensin-Converting-Enzyms aus Benazepril, Enalapril, Ramipril, Quinapril, Perindopril, Lisinopril, Imidapril, Zofenopril, Trandolapril sowie deren Salzen ausgewählt sind.

10. Verfahren nach Anspruch 8, **dadurch gekennzeichnet, dass** die Angiotensin-II-Rezeptor-Antagonisten aus Valsartan, Telmisartan, Losartan, Irbesartan, Azilsartan und Omesartan sowie deren Salzen ausgewählt sind.

11. Verfahren nach einem der Ansprüche 1 bis 8, **dadurch gekennzeichnet, dass** der medizinische Wirkstoff Benazepril oder ein Salz davon in partikulärer Form, vorzugsweise Benazepril-Hydrochlorid, ist.

12. Orale Nährstoff- und Arzneimittelzusammensetzung zur tierärztlichen Verwendung, umfassend einen Kern aus Alleinfuttermittelextrudat, der mit mindestens einer Fettschicht überzogen ist, wobei die Fettschicht mindestens ein medizinisches Mittel umfasst, wobei das medizinische Mittel (i) mindestens einem Wirkstoff, der in Form einer Lösung oder einer Suspension des Wirkstoffs in einer öligen Flüssigkeit vorkonditioniert ist, oder (ii) mindestens einen Wirkstoff, der in Form wachsartiger Granulate vorkonditioniert ist, umfasst.

13. Orale Nährstoff- und Arzneimittelzusammensetzung zur tierärztlichen Verwendung, **dadurch gekennzeichnet, dass** sie durch das Verfahren nach einem der Ansprüche 1 bis 11 erhalten wird.

14. Zusammensetzung nach einem der Ansprüche 12 und 13 zur Verwendung als Arzneimittel zur tierärztlichen Verwendung.

15. Zusammensetzung nach einem der Ansprüche 12 und 13 zur Verwendung zur Vorbeugung oder Behandlung von Niereninsuffizienz bei einem nicht-menschlichen Säuger.

## Claims

1. Method for obtaining a nutritional and medicinal oral composition for veterinary use comprising the following steps:
a) supplying cores of complete feed extrudate, and
b) coating the cores of complete feed extrudate supplied in step a) with at least one layer of fat comprising at least one medicinal agent, said medicinal agent comprising (i) at least one preconditioned active principle in the form of a solution or of a suspension of said active principle in an oily liquid or (ii) at least one preconditioned active principle in the form of waxy granules, at a temperature below 40°C.

2. Method for obtaining a nutritional and medicinal oral composition for veterinary use comprising the following steps:
a) supplying cores of complete feed extrudate, and
b1) bringing the cores of complete feed extrudate into contact with at least one medicinal agent, said medicinal agent comprising (i) at least one preconditioned active principle in the form of a solution or of a suspension of said active principle in an oily liquid or (ii) at least one preconditioned active principle in the form of waxy granules, and
b2) coating the extrudate cores obtained at the end of step b1) with at least one layer of fat, at a temperature below 40°C.

3. Method according to Claim 1, **characterized in that** step b) comprises the following steps:
b3) obtaining a fat composition comprising at least one medicinal agent, said medicinal agent comprising (i) at least one preconditioned active principle in the form of a solution or of a suspension of said active principle in an oily liquid or (ii) at least one preconditioned active principle in the form of waxy granules, and
b4) coating the extrudate cores obtained at the end of step a) with at least one layer of the fat composition comprising said active principle obtained at the end of step b3), at a temperature below 40°C.

4. Method according to one of Claims 1 to 3, **characterized in that** the layer of fat comprises at least one fat of animal or vegetable origin.

5. Method according to one of Claims 1 to 4, **characterized in that** the active pharmaceutical ingredient is sensitive to moisture.

6. Method according to one of Claims 1 to 5, **characterized in that** the active pharmaceutical ingredient is intended for preventing or treating a disorder or a chronic disease.

7. Method according to one of Claims 1 to 6, **characterized in that** the active pharmaceutical ingredient is intended for preventing or treating renal insufficiency.

8. Method according to one of Claims 1 to 7, **characterized in that** the moisture-sensitive active pharmaceutical ingredient is selected from angiotensin-converting enzyme inhibitors (ACE inhibitors), renin inhibitors and angiotensin II receptor antagonists, firocoxib, ciclosporin, S-adenosyl-methionine, eplerenone, spironolactone, amlodipine and levosimendan.

9. Method according to Claim 8, **characterized in that** the angiotensin-converting enzyme inhibitors are selected from benazepril, enalapril, ramipril, quinapril, perindopril, lisinopril, imidapril, zofenopril, trandolapril, as well as salts thereof.

10. Method according to Claim 8, **characterized in that** the angiotensin II receptor antagonists are selected from valsartan, telmisartan, losartan, irbesartan, azilsartan and omesartan, as well as salts thereof.

11. Method according to one of Claims 1 to 8, **characterized in that** the active pharmaceutical ingredient is benazepril or a salt thereof, in particulate form, preferably benazepril hydrochloride.

12. Nutritional and medicinal oral composition for veterinary use comprising a core of complete feed extrudate coated with at least one layer of fat, said layer of fat comprising at least one medicinal agent, said medicinal agent comprising (i) at least one preconditioned active principle in the form of a solution or of a suspension of said active principle in an oily liquid or (ii) at least one preconditioned active principle in the form of waxy granules.

13. Nutritional and medicinal oral composition for veterinary use, **characterized in that** it is obtained by the method according to one of Claims 1 to 11.

14. Composition according to one of Claims 12 and 13, for use as a medicinal product for veterinary use.

15. Composition according to either of Claims 12 and 13, for use for preventing or treating renal insufficiency in a nonhuman mammal.
